(19) Europäisches Patentamt
European Patent Office
Office européen des brevets

(11) **EP 3 151 860 B1**

(12) **EUROPEAN PATENT SPECIFICATION**

(45) Date of publication and mention
of the grant of the patent:
**24.03.2021 Bulletin 2021/12**

(51) Int Cl.:
*A61K 41/00* (2020.01)     *A61K 8/90* (2006.01)
*A61K 8/04* (2006.01)       *A61K 8/49* (2006.01)
*A61K 8/73* (2006.01)       *A61K 8/22* (2006.01)
*A61Q 19/08* (2006.01)      *A61P 17/02* (2006.01)
*A61P 17/06* (2006.01)      *A61P 17/10* (2006.01)
*A61K 31/77* (2006.01)      *A61K 31/352* (2006.01)
*A61K 9/06* (2006.01)       *A61K 9/00* (2006.01)
*A61K 47/34* (2017.01)      *A61N 5/06* (2006.01)
*A61N 5/067* (2006.01)

(21) Application number: **15805894.1**

(22) Date of filing: **09.06.2015**

(86) International application number:
**PCT/CA2015/050530**

(87) International publication number:
**WO 2015/188271 (17.12.2015 Gazette 2015/50)**

(54) **THERMOSETTING BIOPHOTONIC COMPOSITIONS AND USES THEREOF**

WÄRMEHÄRTENDE BIOPHOTONISCHE ZUSAMMENSETZUNGEN UND VERWENDUNGEN DAVON

COMPOSITIONS BIOPHOTONIQUES THERMODURCISSABLES ET UTILISATIONS CORRESPONDANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2014 US 201462009869 P**

(43) Date of publication of application:
**12.04.2017 Bulletin 2017/15**

(73) Proprietor: **Klox Technologies Inc.**
**Laval, QC H7V 4A7 (CA)**

(72) Inventors:
• **PIERGALLINI, Remigio**
**63038 Grottammare (IT)**
• **LOUPIS, Nikolaos**
**14562 Athens (GR)**
• **DEVEMY, Emmanuelle**
**Montréal, Québec H3X 2K6 (CA)**
• **DESROSIERS, Eric**
**Outremont, Québec H2V 1Y7 (CA)**
• **CHENITE, Abdellatif**
**Kirkland, Québec H9H 4L5 (CA)**

(74) Representative: **BCF Global**
**Centre d'Entreprise et d'Innovation**
**56, Bd Niels Bohr**
**CS 52132**
**69603 Villeurbanne Cedex (FR)**

(56) References cited:
**WO-A1-2013/155620     WO-A1-2015/184551**
**CA-A1- 2 408 323**

• **CARLOS VAN HEMELRIJCK ET AL: "Rheological characterization and permeation behavior of poloxamer 407-based systems containing 5-aminolevulinic acid for potential application in photodynamic therapy", INTERNATIONAL JOURNAL OF PHARMACEUTICS, vol. 437, no. 1-2, 1 November 2012 (2012-11-01), pages 120-129, XP055419977, AMSTERDAM, NL ISSN: 0378-5173, DOI: 10.1016/j.ijpharm.2012.07.048**

**(Cont. next page)**

- **GILLES DUMORTIER ET AL: "A Review of Poloxamer 407 Pharmaceutical and Pharmacological Characteristics", PHARMACEUTICAL RESEARCH, KLUWER ACADEMIC PUBLISHERS-PLENUM PUBLISHERS, NL, vol. 23, no. 12, 11 November 2006 (2006-11-11), pages 2709-2728, XP019453318, ISSN: 1573-904X, DOI: 10.1007/S11095-006-9104-4**
- **ESCOBAR-CHAVEZ ET AL.: 'Applications of thermoreversible pluronic F-127 gels in pharmaceutical formulations' J. PHARM. PHARMACEUT. SCI. vol. 9, no. 3, 2006, pages 339 - 358, XP002415851**
- **GRÜNING ET AL.: 'Physicochemical characterisation of a novel thermogelling formulation for percutaneous penetration of 5-aminolevulinic acid' J. PHARMACEUT. SCI. vol. 97, no. 6, 2008, pages 2311 - 2323, XP055242319**
- **COLLAUD ET AL.: 'Thermosetting gel for the delivery of 5-aminolevulinic acid esters to the cervix' J. PHARMACEUT. SCI. vol. 97, no. 7, 2008, pages 2680 - 2690, XP055134376**
- **BOURRE ET AL.: 'Potential efficacy of a delta 5-aminolevulinic acid thermosetting gel formulation for use in photodynamic therapy of lesions of the gastrointestinal tract' PHARMACAL. RES. vol. 45, no. 2, 2002, pages 159 - 165, XP055242322**

**Description**

## BACKGROUND OF THE DISCLOSURE

Phototherapy has recently been recognized as having wide range of applications in both the medical and cosmetic fields including use in surgery, therapy and diagnostics. For example, phototherapy has been used to treat cancers and tumors with lessened invasiveness, to disinfect target sites as an antimicrobial treatment, to promote wound healing, and for facial skin rejuvenation (i.e. WO 2013/155620).

Photodynamic therapy is a type of phototherapy involving the application of a photosensitive agent to target tissue then exposing the target tissue to a light source after a determined period of time during which the photosensitizer is absorbed by the target tissue. Such regimens, however, are often associated with undesired side-effects, including systemic or localized toxicity to the patient or damage to non-targeted tissue. Moreover, such existing regimens often demonstrate low therapeutic efficacy due to, for example, the poor selectivity of the photosensitive agents into the target tissues.

It is an object of the disclosure to provide improved compositions, compositions for their use in therapy and methods for cosmetic treatment with the composition.

## SUMMARY OF THE DISCLOSURE

The present disclosure provides thermosetting biophotonic compositions, compositions for their use in therapy and methods useful in the cosmetic treatment of a target tissue.

The thermosetting biophotonic composition of the invention comprises a block copolymer at a concentration of more than 20% weight per volume of the composition, the block copolymer comprising at least one sequence of polyethylene glycol-propylene glycol (PEG-PPG), polyethylene glycol-poly(lactic acid) (PEG-PLA) or polyethylene glycol-poly($\varepsilon$-caprolacone) (PEG-PCL), and at least one xanthene dye selected from Eosin Y, Erythrosine B, Fluorescein, Rose Bengal and Phloxin B, solubilized within the block copolymer, the at least xanthene dye being from 0.001% and 3% per weight of the composition, the thermosetting biophotonic composition being in liquid form at 22°C and thermosetting on exposure of heat.

Partticularly the block copolymer comprises at least one sequence of polyethylene glycol-propylene glycol (PEG-PPG). More particularly the block copolymer is a poloxamer.

In a particular embodiment, the thermosetting biophotonic composition of the invention further comprises a stabilizer selected from gelatin, hydroxyethyl cellulose (HEC), carboxymethyl cellulose (CMC) and a thickening agent.

In a particular embodiment, the thermosetting biophotonic composition of the invention further comprises an oxidizing agent, wherein the oxidizing agent is selected from hydrogen peroxide, urea peroxide and benzoyl peroxide.

The present disclosure also provides for a method for cosmetic treatment of tissue, the method comprising: applying thermosetting biophotonic composition of the invention over a target skin tissue; and illuminating the thermosetting biophotonic composition with light having a wavelength that overlaps with the absorption spectrum of the at least one xanthene dye.

The cosmetic treatment of tissue may particularly be selected from skin rejuvenation, skin conditioning and promotion of collagen synthesis

The present disclosure also provides for a thermosetting biophotonic composition for use in one of more of

- treating a skin disorder; and
- promoting would healing.

Particularly the skin disorder may be selected from acne, eczema, psoriasis and dermatitis.

## BRIEF DESCRIPTION OF THE DRAWINGS

Further aspects and advantages of the present invention will become better understood with reference to the description in association with the following in which:

Figure 1 illustrates the light emission spectra of a thermosetting biophotonic composition, according to one embodiment of the present disclosure, during 0-5 minutes of illumination.

Figure 2 shows the collagen production in TGF-beta stimulated DHF cells after illumination with the biophotonic thermogel of Figure 1, according to one embodiment of the present disclosure.

## DETAILED DESCRIPTION

*(1) Overview*

The present disclosure provides thermosetting biophotonic membranes and uses thereof. Biophotonic therapy using these compositions would combine the beneficial effects of thermosetting compositions with the photobiostimulation induced by the fluorescent light generated upon illumination of the composition. Furthermore, in certain embodiments, phototherapy using the thermosetting biophotonic compositions of the present disclosure will for instance rejuvenate the skin by, e.g., promoting collagen synthesis; promote wound healing or tissue repair; prevent or treat scars; treat skin conditions such as acne, eczema, psoriasis or dermatitis; or treat periodontitis.

*(2) Definitions*

Before continuing to describe the present disclosure in further detail, it is to be understood that this disclosure is not limited to specific compositions or process steps, as such may vary. It must be noted that, as used in this specification and the appended claims, the singular form "a", "an" and "the" include plural referents unless the context clearly dictates otherwise.

As used herein, the term "about" in the context of a given value or range refers to a value or range that is within 20%, preferably within 10%, and more preferably within 5% of the given value or range.

It is convenient to point out here that "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

"Biophotonic" means the generation, manipulation, detection and application of photons in a biologically relevant context. In other words, biophotonic compositions exert their physiological effects primarily due to the generation and manipulation of photons.

"Thermosetting" means a liquid composition that can undergo a phase transition to a solid or a semi-solid state (e.g., a gel) spontaneously (e.g. on contact with a target tissue) or following exposure to some form of energy (e.g., heat energy). The heat energy may be provided by contact with a warm body, or by a light source. In some embodiments, the phase transition to a solid or semi-sold state can occur on contact with a tissue which is at a higher temperature than the ambient temperature. The terms "thermosetting" and "thermogelling" are used herein interchangeably. The terms "thermoset" and "thermogel" are also used herein interchangeably.

"Topical application" or "topical uses" means application to body surfaces, such as the skin, mucous membranes, vagina, oral cavity, internal surgical wound sites, and the like.

Terms "chromophore" and "photoactivator" are used herein interchangeably. A chromophore means a chemical compound, when contacted by light irradiation, is capable of absorbing the light. The chromophore readily undergoes photoexcitation and can transfer its energy to other molecules or emit it as light (e.g. fluorescence).

"Photobleaching" or "photobleaches" means the photochemical destruction of a chromophore. A chromophore may fully or partially photobleach.

The term "actinic light" is intended to mean light energy emitted from a specific light source (e.g. lamp, LED, or laser) and capable of being absorbed by matter (e.g. the chromophore or photoactivator). In a preferred embodiment, the actinic light is visible light. The terms "actinic light" and "light" are used herein interchangeably.

"Skin rejuvenation" means a process of reducing, diminishing, retarding or reversing one or more signs of skin aging or generally improving the condition of skin. For instance, skin rejuvenation may include increasing luminosity of the skin, reducing pore size, reducing fine lines or wrinkles, improving thin and transparent skin, improving firmness, improving sagging skin (such as that produced by bone loss), improving dry skin (which might itch), reducing or reversing freckles, age spots, spider veins, reducing or preventing the appearance of rough and leathery skin, fine wrinkles that disappear when stretched, reducing loose skin, or improving a blotchy complexion. According to the present disclosure, one or more of the above conditions may be improved or one or more signs of aging may be reduced, diminished, retarded or even reversed by certain embodiments of the compositions, methods and uses of the present disclosure.

"Wound" means an injury to any tissue, including for example, acute, subacute, delayed or difficult to heal wounds, and chronic wounds. Examples of wounds may include both open and closed wounds. Wounds include, for example, amputations, burns, incisions, excisions, lesions, lacerations, abrasions, puncture or penetrating wounds, surgical wounds, amputations, contusions, hematomas, crushing injuries, ulcers (such as for example pressure, diabetic, venous or arterial), scarring, and wounds caused by periodontitis (inflammation of the periodontium).

Features and advantages of the subject matter hereof will become more apparent in light of the following detailed description of selected embodiments, as illustrated in the accompanying figures. As will be realized, the subject matter disclosed and claimed is capable of modifications in various respects, all without departing from the scope of the claims. Accordingly, the drawings and the description are to be regarded as illustrative in nature, and not as restrictive and the full scope of the subject matter is set forth in the claims.

*(3) Thermosetting biophotonic compositions*

The present disclosure provides, in a broad sense, thermosetting biophotonic compositions and methods of using such compositions. Thermosetting biophotonic compositions can be, in a broad sense, activated by light (e.g., photons) of specific wavelength. A thermosetting biophotonic composition according to various embodiments of the present disclosure contains a block copolymer, with at least one chromophore solubilized in the block copolymer. The chromophore in the thermosetting biophotonic composition may be activated by light, accelerating the dispersion of light energy, which leads to light carrying on a therapeutic effect on its own, and/or to the photochemical activation of other agents contained in the composition (e.g., acceleration in the breakdown process of peroxide (an oxidant or oxidizing agent) when such compound is present in the composition or in contact with the composition, leading to the formation of oxygen radicals, such as singlet oxygen).

When a chromophore absorbs a photon of a certain wavelength, it becomes excited. This is an unstable condition and the molecule tries to return to the ground state, giving away the excess energy. For some chromophores, it is favorable to emit the excess energy as light when returning to the ground state. This process is called fluorescence. The peak wavelength of the emitted fluorescence is shifted towards longer wavelengths compared to the absorption wavelengths due to loss of energy in the conversion process. This is called the Stokes' shift. In the proper environment (e.g., in a biophotonic composition) much of this energy is transferred to the other components of the biophotonic composition or to the treatment site directly.

Without being bound to theory, it is thought that fluorescent light emitted by photoactivated chromophores has therapeutic properties due to its femto-, pico-, or nanosecond emission properties which may be recognized by biological cells and tissues, leading to favourable biomodulation. Furthermore, the emitted fluorescent light has a longer wavelength and hence a deeper penetration into the tissue than the activating light. Irradiating tissue with such a broad range of wavelength, including in some embodiments the activating light which passes through the composition, may have different and complementary effects on the cells and tissues. In other words, chromophores are used in the biophotonic compositions of the present disclosure for therapeutic effect on tissues. In one aspect, and without wishing to be bound to any particular theory, the emitted fluorescent light may have an effect that is mechanical in nature by precipitating a disruption in a flow of electrons that results in a destabilization of a bacterial membrane thereby resulting in a loss of structural integrity of the bacterial membrane. This is a distinct application of these photoactive agents and differs from the use of chromophores as simple stains or as catalysts for photo-polymerization.

The thermosetting biophotonic compositions of the present disclosure may have topical uses. In some embodiments, the thermosetting biophotonic compositions are cohesive once they thermoset. The cohesive nature of these thermosetting biophotonic compositions may provide ease of removal from the site of treatment and hence a faster and less messy treatment. Once thermoset, the cohesive nature of these thermosetting biophotonic compositions may also provide a less messy treatment. In addition the thermosetting biophotonic compositions can limit the contact between the chromopore and the tissue.

The thermosetting compositions of the disclosure are designed to thermoset when they come in contact with target skin tissue. In some embodiments, the thermosetting biophotonic composition thermosets at 32deg Celsius or above. In some embodiments, the composition thermosets within 1 minute at 32deg Celsius. In other embodiments, the composition thermosets within 50 seconds, 40 seconds, 30 seconds, 20 seconds, 10 seconds, 5 seconds, or less than 5 seconds at 32deg Celsius. In other embodiments, the composition thermosets spontaneously on contact with the target tissue.

Individual components of the thermosetting biophotonic compositions of the present disclosure, including chromophores, block copolymers, and other optional ingredients, are detailed below.

(a) Chromophores

Chromophores can be fluorescent compounds (or stains) (also known as "fluorochromes" or "fluorophores"). Other dye groups or dyes (biological and histological dyes, food colorings, carotenoids, and other dyes) can also be used.

Photoactivators can be those that are Generally Regarded As Safe (GRAS). Advantageously, photoactivators which are not well tolerated by the skin or other tissues can be included in the thermosetting biophotonic composition of the present disclosure, as in certain embodiments, the photoactivators are dissolved within the block copolymer.

The thermosetting biophotonic composition of the present disclosure comprises a first chromophore which undergoes partial or complete photobleaching upon application of light. The first chromophore is at least one xanthene dye selected from Eosin Y, Erythrosine B, Fluorescein, Rose Bengal and Phloxin B.

It will be appreciated to those skilled in the art that optical properties of a particular chromophore may vary depending on the chromophore's surrounding medium. Therefore, as used herein, a particular chromophore's absorption and/or emission wavelength (or spectrum) corresponds to the wavelengths (or spectrum) measured in a thermosetting biophotonic composition of the present disclosure.

The thermosetting biophotonic composition disclosed herein may include at least one additional chromophore. Combining chromophores may increase photo-absorption by the combined dye molecules and enhance absorption and photo-biomodulation selectivity. This creates multiple possibilities of generating new photosensitive, and/or selective chromophores mixtures. Thus, in certain embodiments, biophotonic compositions of the disclosure include more than one chromophore. When such multi- chromophore compositions are illuminated with light, energy transfer can occur between the chromophores. This process, known as resonance energy transfer, is a widely prevalent photophysical process through which an excited 'donor' chromophore (also referred to herein as first chromophore) transfers its excitation energy to an 'acceptor' chromophore (also referred to herein as second chromophore). The efficiency and directedness of resonance energy transfer depends on the spectral features of donor and acceptor chromophores. In particular, the flow of energy between chromophores is dependent on a spectral overlap reflecting the relative positioning and shapes of the absorption and emission spectra. More specifically, for energy transfer to occur, the emission spectrum of the donor chromophore must overlap with the absorption spectrum of the acceptor chromophore.

Energy transfer manifests itself through decrease or quenching of the donor emission and a reduction of excited state lifetime accompanied also by an increase in acceptor emission intensity. To enhance the energy transfer efficiency, the donor chromophore should have good abilities to absorb photons and emit photons. Furthermore, the more overlap there is between the donor chromophore's emission spectra and the acceptor chromophore's absorption spectra, the better a donor chromophore can transfer energy to the acceptor chromophore.

In certain embodiments, the thermosetting biophotonic composition of the present disclosure further comprises a second chromophore. In some embodiments, the first chromophore has an emission spectrum that overlaps at least about 80%, 50%, 40%, 30%, 20% or 10% with an absorption spectrum of the second chromophore. In one embodiment, the first chromophore has an emission spectrum that overlaps at least about 20% with an absorption spectrum of the second chromophore. In some embodiments, the first chromophore has an emission spectrum that overlaps at least 1-10%, 5-15%, 10-20%, 15-25%, 20-30%, 25-35%, 30-40%, 35-45%, 50-60%, 55-65% or 60-70% with an absorption spectrum of the second chromophore.

% spectral overlap, as used herein, means the % overlap of a donor chromophore's emission wavelength range with an acceptor chromophore's absorption wavelength rage, measured at spectral full width quarter maximum (FWQM). In some embodiments, the second chromophore absorbs at a wavelength in the range of the visible spectrum. In certain embodiments, the second chromophore has an absorption wavelength that is relatively longer than that of the first chromophore within the range of about 50-250, 25-150 or 10-100 nm.

The first chromophore can be present in an amount of about 0.001-40% per weight of the biophotonic composition. When present, the second chromophore can be present in an amount of about 0.001-40% per weight of the biophotonic composition. In certain embodiments, the first chromophore is present in an amount of about 0.001-3%, 0.001- 0.01%, 0.005-0.1%, 0.1-0.5%, 0.5-2%, 1-5%, 2.5-7.5%, 5-10%, 7.5-12.5%, 10-15%,12.5-17.5%, 15-20%, 17.5-22.5%, 20-25%, 22.5-27.5%, 25-30%, 27.5-32.5%, 30-35%, 32.5-37.5%, or 35-40% per weight of the biophotonic composition. In certain embodiments, the second chromophore is present in an amount of about 0.001-3%, 0.001-0.01%, 0.005-0.1%, 0.1-0.5%, 0.5-2%, 1-5%, 2.5-7.5%, 5-10%, 7.5-12.5%, 10- 15%, 12.5-17.5%, 15-20%, 17.5-22.5%, 20-25%, 22.5-27.5%, 25-30%, 27.5-32.5%, 30- 35%, 32.5-37.5%, or 35-40% per weight of the biophotonic composition. In certain embodiments, the total weight per weight of chromophore or combination of chromophores may be in the amount of about 0.005-1%, 0.05-2%, 1-5%, 2.5-7.5%, 5-10%, 7.5-12.5%, 10-15%, 12.5-17.5%, 15-20%, 17.5-22.5%, 20-25%, 22.5-27.5%, 25-30%, 27.5-32.5%, 30-35%, 32.5-37.5%, or 35-40.001% per weight of the biophotonic composition.

The concentration of the chromophore to be used can be selected based on the desired intensity and duration of the biophotonic activity from the thermosetting biophotonic composition, and on the desired medical or cosmetic effect. For example, some dyes such as xanthene dyes reach a 'saturation concentration' after which further increases in concentration do not provide substantially higher emitted fluorescence. Further increasing the chromophore concentration above the saturation concentration can reduce the amount of activating light passing through the matrix. Therefore, if more fluorescence is required for a certain application than activating light, a high concentration of chromophore can be used. However, if a balance is required between the emitted fluorescence and the activating light, a concentration close to or lower than the saturation concentration can be chosen.

Second chromophores that may be used in the thermosetting biophotonic compositions of the present disclosure include, but are not limited to the following:

*Chlorophyll dyes*

Exemplary chlorophyll dyes include but are not limited to chlorophyll a; chlorophyll b; chlorophyllin; bacteriochlorophyll a; bacteriochlorophyll b; bacteriochlorophyll c; bacteriochlorophyll d; protochlorophyll; protochlorophyll a; amphiphilic chlorophyll derivative 1; and amphiphilic chlorophyll derivative 2.

*Xanthene derivatives*

5 Exemplary xanthene dyes include but are not limited to eosin B, eosin B (4',5'- dibromo,2',7'-dinitr- o-fluorescein, dianion); eosin Y (2',4',5',7'-tetrabromo- fluorescein, dianion); eosin (2',4',5',7'-tetrabromo-fluorescein, dianion); eosin (2',4',5',7'-tetrabromo-fluorescein, dianion) methyl ester; eosin (2',4',5',7'-tetrabromo- fluorescein, monoanion) p-isopropylbenzyl ester; eosin derivative (2',7'-dibromo-fluorescein, dianion); eosin derivative (4',5'-dibromo-fluorescein, dianion); eosin derivative (2',7'-dichloro-fluorescein, dianion); eosin derivative (4',5'-dichloro- fluorescein, dianion); eosin derivative (2',7'-diiodo-fluorescein, dianion); eosin derivative (4',5'-diiodo-fluorescein, dianion); eosin derivative (tribromo-fluorescein, dianion); eosin derivative (2',4',5',7'-tetrachlor- o-fluorescein, dianion); eosin; eosin dicetylpyridinium chloride ion pair; erythrosin B (2',4',5',7'-tetraiodo-fluorescein, dianion); erythrosin; erythrosin dianion; fluorescein dianion; phloxin B (2',4',5',7'-tetrabromo-3,4,5,6-tetrachloro-fluorescein, dianion); phloxin B (tetrachloro-tetrabromo-fluorescein); rose bengal (3,4,5,6-tetrachloro- 2',4',5',7'-tetraiodofluorescein, dianion); pyronin G, pyronin J, pyronin Y; Rhodamine dyes such as rhodamines include 4,5-dibromo-rhodamine methyl ester; 4,5-dibromo- rhodamine n-butyl ester; rhodamine 101 methyl ester; rhodamine 123; rhodamine 6G; rhodamine 6G hexyl ester; tetrabromo-rhodamine 123; and tetramethyl-rhodamine ethyl ester.

*Methylene blue dyes*

Exemplary methylene blue derivatives include but are not limited to I-methyl methylene blue; 1,9-dimethyl methylene blue; methylene blue; methylene violet; bromomethylene violet; 4-iodomethylene violet; 1,9-dimethyl-3-dimethyl-amino-7-diethyl-amino- phenothiazine; and 1,9-dimethyl-3-diethylamino-7-dibutyl-amino-phenot- hiazine.

*Azo dyes*

Exemplary azo (or diazo-) dyes include but are not limited to methyl violet, neutral red, para red (pigment red I), amaranth (Azorubine S), Carmoisine (azorubine, food red 3, acid red 14), allura red AC (FD&C 40), tartrazine (FD&C Yellow 5), orange G (acid orange 10), Ponceau 4R (food red 7), methyl red (acid red 2), and murexide-ammonium purpurate.

In some embodiments, the composition includes Eosin Y as a first chromophore and any one or more of Rose Bengal, Fluorescein, Erythrosine, Phloxine B, chlorophyllin as a second chromophore. It is believed that these combinations have a synergistic effect as they can transfer energy to one another when activated due in part to overlaps or close proximity of their absorption and emission spectra. This transferred energy is then emitted as fluorescence or leads to production of reactive oxygen species. This absorbed and re-emitted light is thought to be transmitted throughout the composition, and also to be transmitted into the site of treatment.

In further embodiments, the composition includes the following synergistic combinations: Eosin Y and Fluorescein; Fluorescein and Rose Bengal; Erythrosine in combination with Eosin Y, Rose Bengal or Fluorescein; Phloxine B in combination with one or more of Eosin Y, Rose Bengal, Fluorescein and Erythrosine. Other synergistic chromophore combinations are also possible.

By means of synergistic effects of the chromophore combinations in the thermosetting biophotonic composition, chromophores which cannot normally be activated by an activating light (such as a blue light from an LED), can be activated through energy transfer from chromophores which are activated by the activating light. In this way, the different properties of photoactivated chromophores can be harnessed and tailored according to the cosmetic or the medical therapy required.

For example, Rose Bengal can generate a high yield of singlet oxygen when activated in the presence of molecular oxygen, however it has a low quantum yield in terms of emitted fluorescent light. Rose Bengal has a peak absorption around 540 nm and so can be activated by green light. Eosin Y has a high quantum yield and can be activated by blue light. By combining Rose Bengal with Eosin Y, one obtains a composition which can emit therapeutic fluorescent light and generate singlet oxygen when activated by blue light. In this case, the blue light photoactivates Eosin Y, which transfers some of its energy to Rose Bengal as well as emitting some energy as fluorescence.

In some embodiments, the chromophore or chromophores are selected such that their emitted fluorescent light, on photoactivation, is within one or more of the green, yellow, orange, red and infrared portions of the electromagnetic spectrum, for example having a peak wavelength within the range of about 490 nm to about 800 nm. In certain embodiments, the emitted fluorescent light has a power density of between 0.005 to about 10 mW/cm$^2$, about 0.5 to about 5 mW/cm$^2$.

(b) <u>Block Copolymer</u>

The thermosetting biophotonic composition of the present disclosure comprises a block copolymer. The block copolymer is present in an amount of more than 20% weight per volume of the total composition. In some embodiments, the block copolymer is present in an amount of at least 21%, 22%, 23%, 24% or 25%. In some embodiments, the block copolymer is present in an amount of 21%, 22%, 23%, 24% or 25%.

The term "block copolymer" as used herein refers to a copolymer comprised of 2 or more blocks (or segments) of different homopolymers. The block copolymer in the thermosetting biophotonic composition of the invention comprises at least one sequence of polyethylene glycol-propylene glycol (PEG-PPG), polyethylene glycol-poly(lactic acid) (PEG-PLA) or polyethylene glycol-poly($\epsilon$-caprolacone) (PEG-PCL).

In certain embodiments of any of the foregoing or following the block copolymer is biocompatible. A polymer is "biocompatible" in that the polymer and degradation products thereof are substantially non-toxic to cells or organisms, including non- carcinogenic and non-immunogenic, and are cleared or otherwise degraded in a biological system, such as an organism (patient) without substantial toxic effect.

In certain embodiments the block copolymer is from a group of tri-block copolymers designated Poloxamers. Poloxamers are A-B-A block copolymers in which the A segment is a hydrophilic polyethylene glycol (PEG) homopolymer and the B segment is hydrophobic polypropylene glycol (PPG) homopolymer. Poloxamers and use in pharmaceutical compositions are disclosed in Van Hemelrijk& al. (International Journal of Pharmmaceutics, 2011, vol. 437, no. 1-2, p. 120-129) and Dumortier & al. (Pharmaceutical Research, 2006, vol. 23, no. 12, p. 2709-2728). PEG is also known as polyethylene oxide (PEO) or polyoxyethylene (POE), depending on its molecular weight. Additionally, PPG is also known as polypropylene oxide (PPO), depending on its molecular weight. Poloxamers are commercially available from BASF Corporation. Poloxamers produce reverse thermal gelatin compositions, i.e., with the characteristic that their viscosity increases with increasing temperature up to a point from which viscosity again decreases. Depending on the relative size of the blocks the copolymer can be a solid, liquid or paste. In certain embodiments of the disclosure the poloxamer is Pluronic® F127 (also known as Poloxamer 407). In some embodiments of thermosetting biophotonic composition of the disclosure comprises Pluronic® F127 in the amount of more than 20% weight per volume of the composition. In other embodiments, the pluronic is present in the amount of at least 21%, 22%, 23%, 24%, or 25%. Other poloxamers can also be used.

Since the PEG blocks contribute hydrophilicity to the polymer, increasing the length of the PEG blocks or the total amount of PEG in the polymer will tend to make the polymer more hydrophilic. Depending on the amounts and proportions of the other components of the polymer, the desired overall hydrophilicity, and the nature and chemical functional groups of any drug or therapeutic agent that may be included in a formulation of the polymer, a skilled person can readily adjust the length (or MW) of the PEG blocks used and/or the total amount of PEG incorporated into the polymer, in order to obtain a polymer having the desired physical and chemical characteristics.

The total amount of PEG in the polymer may be about 80 wt % or less, 75 wt % or less, 70 wt % or less, 65 wt % or less, about 60 wt % or less, about 55 wt % or less, or about 50 wt % or less. In particular embodiments, the total amount of PEG is about 55 wt %, 56 wt %, 57 wt %, 58 wt %, 59 wt %, 60 wt %, 61 wt %, 62 wt %, 63 wt %, 64 wt %, 65 wt %, 66 wt %, 67 wt %, 68 wt %, 69 wt %, or about 70 wt %. Unless otherwise specified, a weight percentage of a particular component of the polymer means that the total weight of the polymer is made up of the specified percentage of monomers of that component. For example, 65 wt % PEG means that 65% of the weight of the polymer is made up of PEG monomers, which monomers are linked into blocks of varying lengths, which blocks are distributed along the length of polymer, including in a random distribution.

The presence of surfactants can enhance the fluorescence of the chromophore. However, complimentary surfactant and chromophore combinations can be tested and selected based on non-electrically repelling combinations. For example, a negatively charged chromophore can be used with an ionic or non-ionic surfactant, and vice versa.

The block copolymer may also be mixed with thickening agents or stabilizers such as gelatin and/or modified celluloses such as hydroxyethyl cellulose (HEC) and carboxymethyl cellulose (CMD), and/or polysaccharides such as xanthan gum, guar gum, and/or starches and/or any other thickening agent. In certain embodiments of the disclosure, the stabilizer or thickening agent may comprise gelatin. For example, the surfactant phase may comprise about 0-5 wt%, about 5-25 wt%, about 0-15 wt%, or about 10-20 wt% gelatin.

Thickening agents and/or stabilizers may be selected according to effects they will have on the optical transparency of the biophotonic membrane or the stabilizing effect on the block copolymer. The thermosetting biophotonic composition should be able to transmit sufficient light to activate the at least one chromophore.

(c) Oxidants/Antimicrobials

According to certain embodiments, the thermosetting biophotonic composition of the present disclosure may optionally comprise one or more additional components, such as oxygen-rich compounds as a source of oxygen radicals ("oxidants"). Peroxide compounds are oxidants that contain the peroxy group (R-O-O-R), which is a chainlike structure containing two oxygen atoms, each of which is bonded to the other and a radical or some element. When a thermosetting biophotonic composition of the present disclosure is illuminated with light, the chromophores are excited to a higher energy state. When the chromophores' electrons return to a lower energy state, they emit photons with a lower energy level, thus causing the emission of light of a longer wavelength (Stokes' shift). In the proper environment, some of this energy is transferred to the oxidant, if present, such as a peroxide or oxygen and can cause the formation of oxygen radicals, such as singlet oxygen. The singlet oxygen and other reactive oxygen species generated by the activation of the biophotonic composition are thought to operate in a hormetic fashion. That is, a health beneficial effect that is brought about by the low exposure to a normally toxic stimuli (e.g. reactive oxygen), by stimulating and modulating stress response pathways in cells of the targeted tissues. Endogenous response to exogenous generated free radicals (reactive oxygen species) is modulated in increased defense capacity against the exogenous free radicals and induces acceleration of healing and regenerative processes. Furthermore, this may also produce an antibacterial effect. The extreme sensitivity of bacteria to exposure to free radicals makes the thermosetting biophotonic composition of the present disclosure potentially a bactericidal composition.

Antimicrobials kill microbes or inhibit their growth or accumulation, and are optionally included in the thermosetting biophotonic composition of the present disclosure. Suitable antimicrobials for use in the methods and compositions of the present disclosure include, but not limited to, hydrogen peroxide, urea hydrogen peroxide, benzoyl peroxide, phenolic and chlorinated phenolic and chlorinated phenolic compounds, resorcinol and its derivatives, bisphenolic compounds, benzoic esters (parabens), halogenated carbonilides, polymeric antimicrobial agents, thazolines, trichloromethylthioimides, natural antimicrobial agents (also referred to as "natural essential oils"), metal salts, and broad-spectrum antibiotics.

Hydrogen peroxide ($H_2O_2$) is a powerful oxidizing agent, and breaks down into water and oxygen and does not form any persistent, toxic residual compound. A suitable range of concentration over which hydrogen peroxide can be used in the biophotonic composition is from about 0.1% to about 3%, about 0.1 to 1.5%, about 0.1% to about 1%, about 1%, less than about 1%.

Urea peroxide (also known as carbamide peroxide or percarbamide) is soluble in water and contains approximately 35% hydrogen peroxide. A suitable range of concentration over which urea peroxide can be used in the biophotonic composition of the present disclosure is less than about 0.25 %, or less than about 0.3%, from 0.001 to 0.25%, or from about 0.3% to about 5%. Urea peroxide breaks down to urea and hydrogen peroxide in a slow-release fashion that can be accelerated with heat or photochemical reactions.

Benzoyl peroxide consists of two benzoyl groups (benzoic acid with the H of the carboxylic acid removed) joined by a peroxide group. It is found in treatments for acne, in concentrations varying from 2.5% to 10%. The released peroxide groups are effective at killing bacteria. Benzoyl peroxide also promotes skin turnover and clearing of pores, which further contributes to decreasing bacterial counts and reduce acne. Benzoyl peroxide breaks down to benzoic acid and oxygen upon contact with skin, neither of which is toxic. A suitable range of concentration over which benzoyl peroxide can be used in the thermosetting biophotonic composition is from about 2.5% to about 5%.

Specific phenolic and chlorinated phenolic antimicrobial agents that can be used in the disclosure include, but are not limited to: phenol; 2-methyl phenol; 3-methyl phenol; 4-methyl phenol; 4-ethyl phenol; 2,4-dimethyl phenol; 2,5-dimethyl phenol; 3,4-dimethyl phenol; 2,6-dimethyl phenol; 4-n-propyl phenol; 4-n-butyl phenol; 4-n-amyl phenol; 4-tert-amyl phenol; 4-n-hexyl phenol; 4-n-heptyl phenol; mono- and poly-alkyl and aromatic halophenols; p-chlorophenyl; methyl p-chlorophenol; ethyl p-chlorophenol; n-propyl p-chlorophenol; n-butyl p-chlorophenol; n-amyl p-chlorophenol; sec-amyl p-chlorophenol; n-hexyl p-chlorophenol; cyclohexyl p-chlorophenol; n-heptyl p-chlorophenol; n-octyl; p-chlorophenol; o-chlorophenol; methyl o-chlorophenol; ethyl o-chlorophenol; n-propyl o-chlorophenol; n-butyl o-chlorophenol; n-amyl o-chlorophenol; tert-amyl o-chlorophenol; n-hexyl o-chlorophenol; n-heptyl o-chlorophenol; o-benzyl p-chlorophenol; o-benxyl-m-methyl p-chlorophenol; o-benzyl-m,m-dimethyl p-chlorophenol; o-phenylethyl p-chlorophenol; o-phenylethyl-m-methyl p-chlorophenol; 3-methyl p-chlorophenol 3,5-dimethyl p-chlorophenol, 6-ethyl-3-methyl p-chlorophenol, 6-n-propyl-3-methyl p-chlorophenol; 6-iso-propyl-3-methyl p-chlorophenol; 2-ethyl-3,5-dimethyl p-chlorophenol; 6-sec-butyl-3-methyl p-chlorophenol; 2-iso-propyl-3,5-dimethyl p-chlorophenol; 6-diethylmethyl-3-methyl p-chlorophenol; 6-iso-propyl-2-ethyl-3-methyl p-chlorophenol; 2-sec-amyl-3,5-dimethyl p-chlorophenol; 2-diethylmethyl-3,5-dimethyl p-chlorophenol; 6-sec-octyl-3-methyl p-chlorophenol; p-chloro-m-cresol p-bromophenol; methyl p-bromophenol; ethyl p-bromophenol; n-propyl p-bromophenol; n-butyl p-bromophenol; n-amyl p-bromophenol; sec-amyl p-bromophenol; n-hexyl p-bromophenol; cyclohexyl p-bromophenol; o-bromophenol; tert-amyl o-bromophenol; n-hexyl o-bromophenol; n-propyl-m,m-dimethyl o-bromophenol; 2-phenyl phenol; 4-chloro-2-methyl phenol; 4-chloro-3-methyl phenol; 4-chloro-3,5-dimethyl phenol; 2,4-dichloro-3,5-dimethylphenol; 3,4,5,6-tetabromo-2-methylphenol- ; 5-methyl-2-pentylphenol; 4-isopropyl-3-methylphenol; para-chloro-metaxylenol (PCMX); chlorothymol; phenoxyethanol; phenoxyisopropanol; and 5-chloro-2-hydroxydiphenylmethane.

Resorcinol and its derivatives can also be used as antimicrobial agents. Specific resorcinol derivatives include, but are not limited to: methyl resorcinol; ethyl resorcinol; n-propyl resorcinol; n-butyl resorcinol; n-amyl resorcinol; n-hexyl resorcinol; n-heptyl resorcinol; n-octyl resorcinol; n-nonyl resorcinol; phenyl resorcinol; benzyl resorcinol; phenylethyl resorcinol; phenylpropyl resorcinol; p-chlorobenzyl resorcinol; 5-chloro-2,4-dihydroxydiphenyl methane; 4'-chloro-2,4-dihydroxydiphenyl methane; 5-bromo-2,4-dihydroxydiphenyl methane; and 4'-bromo-2,4-dihydroxydiphenyl methane.

Specific bisphenolic antimicrobial agents that can be used in the disclosure include, but are not limited to: 2,2'-methylene bis-(4-chlorophenol); 2,4,4'trichloro-2'-hydroxydiphenyl ether, which is sold by Ciba Geigy, Florham Park, N.J. under the tradename Triclosan®; 2,2'-methylene bis-(3,4,6-trichlorophenol); 2,2'-methylene bis-(4-chloro-6-bromophenol); bis-(2-hydroxy-3,5-dichlorop- henyl) sulphide; and bis-(2-hydroxy-5-chlorobenzyl)sulphide.

Specific benzoie esters (parabens) that can be used in the disclosure include, but are not limited to: methylparaben; propylparaben; butylparaben; ethylparaben; isopropylparaben; isobutylparaben; benzylparaben; sodium methylparaben; and sodium propylparaben.

Specific halogenated carbanilides that can be used in the disclosure include, but are not limited to: 3,4,4'-trichlorocarbanilides, such as 3-(4-chlorophenyl)-1-(3,4-dichlorphenyl)urea sold under the tradename Triclocarban® by Ciba-Geigy, Florham Park, N.J.; 3-trifluoromethyl-4,4'-dichlorocarbanilide; and 3,3',4-trichlorocarbanilide. Specific polymeric antimicrobial agents that can be used in the disclosure include, but are not limited to: polyhexamethylene biguanide hydrochloride; and poly(iminoimidocarbonyl iminoimidocarbonyl iminohexamethylene hydrochloride), which is sold under the tradename Vantocil® IB.

Specific thazolines that can be used in the disclosure include, but are not limited to that sold under the tradename Micro-Check®; and 2-n-octyl-4-isothiazolin-3-one, which is sold under the tradename Vinyzene® IT-3000 DIDP.

Specific trichloromethylthioimides that can be used in the disclosure include, but are not limited to: N-(trichloromethylthio)phthalimide, which is sold under the tradename Fungitrol®; and N-trichloromethylthio-4-cyclohexene-1,2-dicarboximide, which is sold under the tradename Vancide®.

Specific natural antimicrobial agents that can be used in the disclosure include, but are not limited to, oils of: anise; lemon; orange; rosemary; wintergreen; thyme; lavender; cloves; hops; tea tree; citronella; wheat; barley; lemongrass; cedar leaf; cedarwood; cinnamon; fleagrass; geranium; sandalwood; violet; cranberry; eucalyptus; vervain; peppermint; gum benzoin; basil; fennel; fir; balsam; menthol; ocmea origanuin; hydastis; carradensis; Berberidaceac daceae; Ratanhiae longa; and Curcuma longa. Also included in this class of natural antimicrobial agents are the key chemical components of the plant oils which have been found to provide antimicrobial benefit. These chemicals include, but are not limited to: anethol; catechole; camphene; thymol; eugenol; eucalyptol; ferulic acid; farnesol; hinokitiol; tropolone; limonene; menthol; methyl salicylate; carvacol; terpineol; verbenone; berberine; ratanhiae extract; caryophellene oxide; citronellic acid; curcumin; nerolidol; and geraniol.

Specific metal salts that can be used in the disclosure include, but are not limited to, salts of metals in groups 3a-5a, 3b-7b, and 8 of the periodic table. Specific examples of metal salts include, but are not limited to, salts of: aluminum; zirconium; zinc; silver; gold; copper; lanthanum; tin; mercury; bismuth; selenium; strontium; scandium; yttrium; cerium; praseodymiun; neodymium; promethum; samarium; europium; gadolinium; terbium; dysprosium; holmium; erbium; thalium; ytterbium; lutetium; and mixtures thereof. An example of the metal-ion based antimicrobial agent is sold under the tradename HealthShield®, and is manufactured by HealthShield Technology, Wakefield, Mass.

Specific broad-spectrum antimicrobial agents that can be used in the disclosure include, but are not limited to, those that are recited in other categories of antimicrobial agents herein.

Additional antimicrobial agents that can be used in the methods of the disclosure include, but are not limited to: pyrithiones, and in particular pyrithione-including zinc complexes such as that sold under the tradename Octopirox®; dimethyidimethylol hydantoin, which is sold under the tradename Glydant®; methylchloroisothiazolinone/methylisothiazolinone, which is sold under the tradename Kathon CG®; sodium sulfite; sodium bisulfite; imidazolidinyl urea, which is sold under the tradename Germall 115®; diazolidinyl urea, which is sold under the tradename Germall 11®; benzyl alcohol v2-bromo-2-nitropropane-1,3-diol, which is sold under the tradename Bronopol®; formalin or formaldehyde; iodopropenyl butylcarbamate, which is sold under the tradename Polyphase P100®; chloroacetamide; methanamine; methyldibromonitrile glutaronitrile (1,2-dibromo-2,4-dicyanobutane), which is sold under the tradename Tektamer®; glutaraldehyde; 5-bromo-5-nitro-1,3-dioxane, which is sold under the tradename Bronidox®; phenethyl alcohol; o-phenylphenol/sodium o-phenylphenol sodium hydroxymethylglycinate, which is sold under the tradename Suttocide A®; polymethoxy bicyclic oxazolidine; which is sold under the tradename Nuosept C®; dimethoxane; thimersal; dichlorobenzyl alcohol; captan; chlorphenenesin; dichlorophene; chlorbutanol; glyceryl laurate; halogenated diphenyl ethers; 2,4,4'-trichloro-2'-hydroxy-diphenyl ether, which is sold under the tradename Triclosan® and is available from Ciba-Geigy, Florham Park, N.J.; and 2,2'-dihydroxy-5,5'-dibromo-diphenyl ether.

*(4) Optical properties of the Thermosetting Biophotonic Compositions*

In certain embodiments, biophotonic compositions of the present disclosure are substantially transparent or translucent. The % transmittance of the biophotonic composition can be measured in the range of wavelengths from 250 nm to 800 nm using, for example, a Perkin-Elmer Lambda 9500 series UV-visible spectrophotometer. In some embodiments, transmittance within the visible range is measured and averaged. In some other embodiments, transmittance of the thermosetting biophotonic composition is measured with the chromophore omitted. As transmittance is dependent upon thickness, the thickness of each sample can be measured with calipers prior to loading in the spectrophotometer. Transmittance values can be normalized according to

$$F_{T-corr}(\lambda, t_2) = [e^{-\sigma_t(\lambda)t_1}]^{\frac{t_2}{t_1}} = [F_{T-corr}(\lambda, t_1)]^{\frac{t_2}{t_1}},$$

where $t_1$=actual specimen thickness, $t_2$=thickness to which transmittance measurements can be normalized. In the art, transmittance measurements are usually normalized to 1 cm.

In some embodiments, the biophotonic composition has a transmittance that is more than about 20%, 30%, 40%, 50%, 60%, 70%, or 75% within the visible range. In some embodiments, the transmittance exceeds 40%, 41%, 42%, 43%, 44%, or 45% within the visible range. In some embodiments, the composition has a light transmittance of about 40-100%, 45-100%, 50-100%, 55-100%, 60-100%, 65-100%, 70-100%, 75-100%, 80- 100%, 85-100%, 90-100%, or 95-100%.

*(5) Applications*

The thermosetting biophotonic composition of the present disclosure may have cosmetic and/or medical benefits. They can be used to promote skin rejuvenation and skin conditioning, promote the treatment of a skin disorder such as acne, eczema, dermatitis or psoriasis, promote tissue repair, promote wound healing including periodontal pockets, prevent or treat scarring, prevent or treat bacterial, fungal or viral infections. They can be used to treat acute inflammation. Acute inflammation can present itself as pain, heat, redness, swelling and loss of function. It includes those seen in allergic reactions such as insect bites e.g.; mosquito, bees, wasps, poison ivy, or post-ablative treatment.

With the compositions of the present disclosure, any source of actinic light can be used. Any type of halogen, LED or plasma arc lamp, or laser may be suitable. The primary characteristic of suitable sources of actinic light will be that they emit light in a wavelength (or wavelengths) appropriate for activating the one or more photoactivators present in the composition. In one embodiment, an argon laser is used. In another embodiment, a potassium-titanyl phosphate (KTP) laser (e.g. a GreenLight™ laser) is used. In yet another embodiment, a LED lamp such as a photocuring device is the source of the actinic light. In yet another embodiment, the source of the actinic light is a source of light having a wavelength between about 200 to 800 nm. In another embodiment, the source of the actinic light is a source of visible light having a wavelength between about 400 and 600 nm. In another embodiment, the source of the actinic light is a source of visible light having a wavelength between about 400 and 800 nm. In another embodiment, the source of the actinic light is a source of visible light having a wavelength between about 400 and 700 nm. In yet another embodiment, the source of the actinic light is blue light. In yet another embodiment, the source of the actinic light is red light. In yet another embodiment, the source of the actinic light is green light. Furthermore, the source of actinic light should have a suitable power density. Suitable power density for non-collimated light sources (LED, halogen or plasma lamps) are in the range from about 0.1 mW/cm$^2$ to about 200 mW/cm$^2$• Suitable power density for laser light sources are in the range from about 0.5 mW/cm$^2$ to about 0.8 mW/cm$^2$.

Generally, the light has an energy at the subject's skin surface of between about 0.1 mW/cm$^2$ and about 500 mW/cm$^2$ or 0.1-300 mW/cm$^2$ , or 0.1-200 mW/cm$^2$, wherein the energy applied depends at least on the condition being treated, the wavelength of the light, the distance of the skin from the light source and the thickness of the biophotonic composition applied to the target skin or wound. In certain embodiments, the light at the subject's skin is between about 1-40 mW/cm$^2$, or 20-60 mW/cm$^2$, or 40-80 mW/cm$^2$, or 60-100 mW/cm$^2$, or 80-120 mW/cm$^2$, or 100-140 mW/cm$^2$, or 30-180 mW/cm$^2$, or 120-160 mW/cm$^2$, or 140-180 mW/cm$^2$, or 160-200 mW/cm$^2$, or 110-240 mW/cm$^2$, or 110-150 mW/cm$^2$, or 190-240 mW/cm$^2$.

The activation of the chromophore(s) within the biophotonic composition may take place almost immediately on illumination (femto- or pico seconds). A prolonged exposure period may be beneficial to exploit the synergistic effects of the absorbed, reflected and reemitted light of the biophotonic composition of the present disclosure and its interaction with the tissue being treated. The time of exposure of the tissue or skin or biophotonic composition to actinic light may be a period between .01 minutes and 90 minutes. In another embodiment, the time of exposure of the tissue or skin or biophotonic composition to actinic light is a period between 1 minute and 5 minutes. In some other embodiments, the biophotonic composition is illuminated for a period between 1 minute and 3 minutes. In certain embodiments, light is applied for a period of 1-30 seconds, 15-45 seconds, 30-60 seconds, 0.75-1.5 minutes, 1-2 minutes, 1.5-2.5 minutes, 2-3 minutes, 2.5-3.5 minutes, 3-4 minutes, 3.5-4.5 minutes, 4-5 minutes, 5-10 minutes, 10-15 minutes, 15-20 minutes, or 20-30 minutes. The treatment time may range up to about 90 minutes, about 80 minutes, about 70 minutes, about 60 minutes, about 50 minutes, about 40 minutes or about 30 minutes. It will be appreciated that the treatment time can be adjusted in order to maintain a dosage by adjusting the rate of fluence delivered to a treatment area. For example, the delivered fluence may be about 4 to about 60 J/cm$^2$, about 10 to about 60 J/cm$^2$, 4 to about 90 J/cm$^2$, about 10 to about 90 J/cm$^2$ about 10 to about 50 J/cm$^2$ about 10 to about 40 J/cm$^2$, about 10 to about 30 J/cm$^2$, about 20 to about

40 J/cm$^2$, about 15 J/cm$^2$ to 25 J/cm$^2$, or about 10 to about 20 J/cm$^2$.

The thermosetting biophotonic composition may be reilluminated at certain intervals. In yet another embodiment, the source of actinic light is in continuous motion over the treated area for the appropriate time of exposure. In yet another embodiment, the thermosetting biophotonic composition may be illuminated until the chromophore is at least partially photobleached or fully photobleached.

The chromophore(s) can be photoexcited by ambient light including from the sun and overhead lighting. In certain embodiments, the chromophore(s) can be photoactivated by light in the visible range of the electromagnetic spectrum. The light can be emitted by any light source such as sunlight, light bulb, an LED device, electronic display screens such as on a television, computer, telephone, mobile device, flashlights on mobile devices. In the methods of the present disclosure, any source of light can be used. For example, a combination of ambient light and direct sunlight or direct artificial light may be used. Ambient light can include overhead lighting such as LED bulbs, fluorescent bulbs etc, and indirect sunlight.

The thermosetting biophotonic composition may be removed from the skin following application of light. Otherwise, the thermosetting biophotonic composition is left on the tissue for an extended period of time and reactivated with direct or ambient light at appropriate times to treat the condition.

The thermosetting biophotonic composition can be applied to the tissue, such as on the face, once, twice, three times, four times, five times or six times a week, daily, or at any other frequency. The total treatment time can be one week, two weeks, three weeks, four weeks, five weeks, six weeks, seven weeks, eight weeks, nine weeks, ten weeks, eleven weeks, twelve weeks, or any other length of time deemed appropriate. In certain embodiments, the total tissue area to be treated may be split into separate areas (cheeks, forehead), and each area treated separately. For example, the thermosetting biophotonic composition may be applied topically to a first portion of the skin, and that portion illuminated with light, and the composition then removed. Then the biophotonic composition is applied to a second portion of the skin, illuminated and removed. Finally, the biophotonic composition is applied to a third portion, illuminated and removed.

The thermosetting biophotonic composition can be used following wound closure to optimize scar revision. In this case, the thermosetting biophotonic composition may be applied at regular intervals such as once a week, or at an interval deemed appropriate by the physician.

The thermosetting biophotonic composition can be used following acne treatment to maintain the condition of the treated skin. In this case, the composition may be applied at regular intervals such as once a week, or at an interval deemed appropriate by the physician.

The thermosetting biophotonic composition can be used following ablative skin rejuvenation treatment to maintain the condition of the treated skin. In this case, the composition may be applied at regular intervals such as once a week, or at an interval deemed appropriate by the physician.

The thermosetting biophotonic composition can be used to treat eczema or psoriasis. In this case, the composition may be applied at regular intervals such as once a week, or at an interval deemed appropriate by the physician. It may be less painful to the patient to spray the composition onto the affected area.

Additional components may optionally be included in the thermosetting biophotonic composition or used in combination with the compositions. Such additional components include, but are not limited to, healing factors, antimicrobials, oxygen-rich agents, wrinkle fillers such as botox, hyaluronic acid and polylactic acid, fungal, anti-bacterial, anti-viral agents and/or agents that promote collagen synthesis. These additional components may be applied to the skin in a topical fashion, prior to, at the same time of, and/or after topical application of the thermosetting biophotonic compositions of the present disclosure. Suitable healing factors comprise compounds that promote or enhance the healing or regenerative process of the tissues on the application site. During the photoactivation of a thermosetting biophotonic composition of the present disclosure, there may be an increase of the absorption of molecules of such additional components at the treatment site by the skin or the mucosa. In certain embodiments, an augmentation in the blood flow at the site of treatment can observed for a period of time. An increase in the lymphatic drainage and a possible change in the osmotic equilibrium due to the dynamic interaction of the free radical cascades can be enhanced or even fortified with the inclusion of healing factors. Healing factors may also modulate the biophotonic output from the biophotonic composition such as photobleaching time and profile, or modulate leaching of certain ingredients within the composition. Suitable healing factors include, but are not limited to glucosamines, allantoins, saffron, agents that promote collagen synthesis, anti-fungal, anti-bacterial, anti-viral agents and wound healing factors such as growth factors.

(i) Skin Rejuvenation

The thermosetting biophotonic compositions of the present disclosure may be useful in promoting skin rejuvenation or improving skin condition and appearance. The dermis is the second layer of skin, containing the structural elements of the skin, the connective tissue. There are various types of connective tissue with different functions. Elastin fibers give the skin its elasticity, and collagen gives the skin its strength.

The junction between the dermis and the epidermis is an important structure. The dermal-epidermal junction interlocks forming finger-like epidermal ridges. The cells of the epidermis receive their nutrients from the blood vessels in the dermis. The epidermal ridges increase the surface area of the epidermis that is exposed to these blood vessels and the needed nutrients.

The aging of skin comes with significant physiological changes to the skin. The generation of new skin cells slows down, and the epidermal ridges of the dermal-epidermal junction flatten out. While the number of elastin fibers increases, their structure and coherence decreases. Also the amount of collagen and the thickness of the dermis decrease with the ageing of the skin.

Collagen is a major component of the skin's extracellular matrix, providing a structural framework. During the aging process, the decrease of collagen synthesis and insolubilization of collagen fibers contribute to a thinning of the dermis and loss of the skin's biomechanical properties.

The physiological changes to the skin result in noticeable aging symptoms often referred to as chronological-, intrinsic- and photo-aging. The skin becomes drier, roughness and scaling increase, the appearance becomes duller, and most obviously fine lines and wrinkles appear. Other symptoms or signs of skin aging include, but are not limited to, thinning and transparent skin, loss of underlying fat (leading to hollowed cheeks and eye sockets as well as noticeable loss of firmness on the hands and neck), bone loss (such that bones shrink away from the skin due to bone loss, which causes sagging skin), dry skin (which might itch), inability to sweat sufficiently to cool the skin, unwanted facial hair, freckles, age spots, spider veins, rough and leathery skin, fine wrinkles that disappear when stretched, loose skin, a blotchy complexion.

The dermal-epidermal junction is a basement membrane that separates the keratinocytes in the epidermis from the extracellular matrix, which lies below in the dermis. This membrane consists of two layers: the basal lamina in contact with the keratinocytes, and the underlying reticular lamina in contact with the extracellular matrix. The basal lamina is rich in collagen type IV and laminin, molecules that play a role in providing a structural network and bioadhesive properties for cell attachment.

Laminin is a glycoprotein that only exists in basement membranes. It is composed of three polypeptide chains (alpha, beta and gamma) arranged in the shape of an asymmetric cross and held together by disulfide bonds. The three chains exist as different subtypes which result in twelve different isoforms for laminin, including Laminin-1 and Laminin-5.

The dermis is anchored to hemidesmosomes, specific junction points located on the keratinocytes, which consist of a-integrins and other proteins, at the basal membrane keratinocytes by type VII collagen fibrils. Laminins, and particularly Laminin-5, constitute the real anchor point between hemidesmosomal transmembrane proteins in basal keratinocytes and type VII collagen.

Laminin-5 synthesis and type VII collagen expression have been proven to decrease in aged skin. This causes a loss of contact between dermis and epidermis, and results in the skin losing elasticity and becoming saggy.

Recently another type of wrinkles, generally referred to as expression wrinkles, received general recognition. Expression wrinkles result from a loss of resilience, particularly in the dermis, because of which the skin is no longer able to resume its original state when facial muscles which produce facial expressions.

In certain embodiments, the thermosetting biophotonic composition may be used in conjunction with collagen promoting agents. Agents that promote collagen synthesis (i.e., pro-collagen synthesis agents) include amino acids, peptides, proteins, lipids, small chemical molecules, natural products and extracts from natural products.

For instance, it was discovered that intake of vitamin C, iron, and collagen can effectively increase the amount of collagen in skin or bone. See, e.g., U.S. Patent Application Publication 20090069217. Examples of the vitamin C include an ascorbic acid derivative such as L-ascorbic acid or sodium L-ascorbate, an ascorbic acid preparation obtained by coating ascorbic acid with an emulsifier or the like, and a mixture containing two or more of those vitamin Cs at an arbitrary rate. In addition, natural products containing vitamin C such as acerola or lemon may also be used. Examples of the iron preparation include: an inorganic iron such as ferrous sulfate, sodium ferrous citrate, or ferric pyrophosphate; an organic iron such as heme iron, ferritin iron, or lactoferrin iron; and a mixture containing two or more of those irons at an arbitrary rate. In addition, natural products containing iron such as spinach or liver may also be used. Moreover, examples of the collagen include: an extract obtained by treating bone, skin, or the like of a mammal such as bovine or swine with an acid or alkaline; a peptide obtained by hydrolyzing the extract with a protease such as pepsin, trypsin, or chymotrypsin; and a mixture containing two or more of those collagens at an arbitrary rate. Collagens extracted from plant sources may also be used.

(ii) Skin disorders

The thermosetting biophotonic compositions of the present disclosure may be used to treat skin disorders that include, but are not limited to, erythema, telangiectasia, actinic telangiectasia, basal cell carcinoma, contact dermatitis, dermatofibrosarcoma protuberans, genital warts, hidradenitis suppurativa, melanoma, merkel cell carcinoma, nummular dermatitis, molloscum contagiosum, psoriasis, psoriatic arthritis, rosacea, scabies, scalp psoriasis, sebaceous carcinoma, squamous cell carcinoma, seborrheic dermatitis, seborrheic keratosis, shingles, tinea versicolor, warts, skin cancer, pemphigus, sunburn, dermatitis, eczema, rashes, impetigo, lichen simplex chronicus, rhinophyma, perioral dermatitis, pseudofolliculitis barbae, drug eruptions, erythema multiforme, erythema nodosum, granuloma annulare, actinic keratosis, purpura, alopecia areata, aphthous stomatitis, dry skin, chapping, xerosis, ichthyosis vulgaris, fungal infections, herpes simplex, intertrigo, keloids, keratoses, milia, moluscum contagiosum, pityriasis rosea, pruritus, urticaria, and vascular tumors and malformations. Dermatitis includes contact dermatitis, atopic dermatitis, seborrheic dermatitis, nummular dermatitis, generalized exfoliative dermatitis, and statis dermatitis. Skin cancers include melanoma, basal cell carcinoma, and squamous cell carcinoma.

(iii) Acne and Acne Scars

The thermosetting biophotonic compositions of the present disclosure may be used to treat acne. As used herein, "acne" means a disorder of the skin caused by inflammation of skin glands or hair follicles. The thermosetting biophotonic compositions and methods of the disclosure can be used to treat acne at early pre- emergent stages or later stages where lesions from acne are visible. Mild, moderate and severe acne can be treated with embodiments of the biophotonic compositions . Early pre-emergent stages of acne usually begin with an excessive secretion of sebum or dermal oil from the sebaceous glands located in the pilosebaceous apparatus. Sebum reaches the skin surface through the duct of the hair follicle. The presence of excessive amounts of sebum in the duct and on the skin tends to obstruct or stagnate the normal flow of sebum from the follicular duct, thus producing a thickening and solidification of the sebum to create a solid plug known as a comedone. In the normal sequence of developing acne, hyperkeratinazation of the follicular opening is stimulated, thus completing blocking of the duct. The usual results are papules, pustules, or cysts, often contaminated with bacteria, which cause secondary infections. Acne is characterized particularly by the presence of comedones, inflammatory papules, or cysts. The appearance of acne may range from slight skin irritation to pitting and even the development of disfiguring scars. Accordingly, the thermosetting biophotonic compositions of the present disclosure can be used to treat one or more of skin irritation, pitting, development of scars, comedones, inflammatory papules, cysts, hyperkeratinazation, and thickening and hardening of sebum associated with acne.

Some skin disorders present various symptoms including redness, flushing, burning, scaling, pimples, papules, pustules, comedones, macules, nodules, vesicles, blisters, telangiectasia, spider veins, sores, surface irritations or pain, itching, inflammation, red, purple, or blue patches or discolorations, moles, and/or tumors.

The thermosetting biophotonic compositions of the present disclosure may be used to treat various types of acne. Some types of acne include, for example, acne vulgaris, cystic acne, acne atrophica, bromide acne, chlorine acne, acne conglobata, acne cosmetica, acne detergicans, epidemic acne, acne estivalis, acne fulminans, halogen acne, acne indurata, iodide acne, acne keloid, acne mechanica, acne papulosa, pomade acne, premenstral acne, acne pustulosa, acne scorbutica, acne scrofulosorum, acne urticata, acne varioliformis, acne venenata, propionic acne, acne excoriee, gram negative acne, steroid acne, and nodulocystic acne.

The thermosetting biophotonic composition of the present disclosure may be used in conjunction with systemic or topical antibiotic treatment. For example, antibiotics used to treat acne include tetracycline, erythromycin, minocycline, doxycycline, which may also be used with the compositions of the present disclosure. The use of the thermosetting biophotonic composition can reduce the time needed for the antibiotic treatment or reduce the dosage.

(iv) Wound Healing

The thermosetting biophotonic compositions of the present disclosure may be used to treat wounds, promote wound healing. Wounds that may be treated by the biophotonic compositions and methods of the present disclosure include, for example, injuries to the skin and subcutaneous tissue initiated in different ways (e.g., pressure ulcers from extended bed rest, wounds induced by trauma or surgery, bums, ulcers linked to diabetes or venous insufficiency, wounds induced by conditions such as periodontitis) and with varying characteristics. The thermosetting biophotonic compositions may be used for treating and/or promoting the healing of, for example, burns, incisions, excisions, lesions, lacerations, abrasions, puncture or penetrating wounds, surgical wounds, contusions, hematomas, crushing injuries, amputations, sores and ulcers.

The thermosetting biophotonic composition of the present disclosure may be used to treat and/or promote the healing of chronic cutaneous ulcers or wounds, which are wounds that have failed to proceed through an orderly and timely series of events to produce a durable structural, functional, and cosmetic closure. The vast majority of chronic wounds can be classified into three categories based on their etiology: pressure ulcers, neuropathic (diabetic foot) ulcers and vascular (venous or arterial) ulcers.

For example, the thermosetting biophotonic compositions may be used for treating and/or promoting healing of a diabetic ulcer. Diabetic patients are prone to foot and other ulcerations due to both neurologic and vascular complications. Peripheral neuropathy can cause altered or complete loss of sensation in the foot and/or leg. Diabetic patients with advanced neuropathy lose all ability for sharp- dull discrimination. Any cuts or trauma to the foot may go completely unnoticed for days or weeks in a patient with neuropathy. A patient with advanced neuropathy loses the ability to sense a sustained pressure insult, as a result, tissue ischemia and necrosis may occur leading to for example, plantar ulcerations. Microvascular disease is one of the significant complications for diabetics which may also lead to ulcerations. The thermosetting biophotonic compositions may be used for treating a chronic wound, where the chronic wound is characterized by diabetic foot ulcers and/or ulcerations due to neurologic and/or vascular complications of diabetes.

In other examples, the present thermosetting biophotonic compositions may be used for treating and/or promoting healing of a pressure ulcer. Pressure ulcers include bed sores, decubitus ulcers and ischial tuberosity ulcers and can cause considerable pain and discomfort to a patient. A pressure ulcer can occur as a result of a prolonged pressure applied to the skin. Thus, pressure can be exerted on the skin of a patient due to the weight or mass of an individual. A pressure ulcer can develop when blood supply to an area of the skin is obstructed or cut off for more than two or three hours. The affected skin area can turn red, become painful and necrotic. If untreated, the skin can break open and become infected. A pressure ulcer is therefore a skin ulcer that occurs in an area of the skin that is under pressure from e.g. lying in bed, sitting in a wheelchair, and/or wearing a cast for a prolonged period of time. Pressure ulcers can occur when a person is bedridden, unconscious, unable to sense pain, or immobile. Pressure ulcers often occur in boney prominences of the body such as the buttocks area (on the sacrum or iliac crest), or on the heels of foot.

There are three distinct phases in the wound healing process. First, in the inflammatory phase, which typically occurs from the moment a wound occurs until the first two to five days, platelets aggregate to deposit granules, promoting the deposit of fibrin and stimulating the release of growth factors. Leukocytes migrate to the wound site and begin to digest and transport debris away from the wound. During this inflammatory phase, monocytes are also converted to macrophages, which release growth factors for stimulating angiogenesis and the production of fibroblasts.

Second, in the proliferative phase, which typically occurs from two days to three weeks, granulation tissue forms, and epithelialization and contraction begin. Fibroblasts, which are key cell types in this phase, proliferate and synthesize collagen to fill the wound and provide a strong matrix on which epithelial cells grow. As fibroblasts produce collagen, vascularization extends from nearby vessels, resulting in granulation tissue. Granulation tissue typically grows from the base of the wound. Epithelialization involves the migration of epithelial cells from the wound surfaces to seal the wound. Epithelial cells are driven by the need to contact cells of like type and are guided by a network of fibrin strands that function as a grid over which these cells migrate. Contractile cells called myofibroblasts appear in wounds, and aid in wound closure. These cells exhibit collagen synthesis and contractility, and are common in granulating wounds.

Third, in the remodeling phase, the final phase of wound healing which can take place from three weeks up to several years, collagen in the scar undergoes repeated degradation and re-synthesis. During this phase, the tensile strength of the newly formed skin increases.

However, as the rate of wound healing increases, there is often an associated increase in scar formation. Scarring is a consequence of the healing process in most adult animal and human tissues. Scar tissue is not identical to the tissue which it replaces, as it is usually of inferior functional quality. The types of scars include, but are not limited to, atrophic, hypertrophic and keloidal scars, as well as scar contractures. Atrophic scars are flat and depressed below the surrounding skin as a valley or hole. Hypertrophic scars are elevated scars that remain within the boundaries of the original lesion, and often contain excessive collagen arranged in an abnormal pattern. Keloidal scars are elevated scars that spread beyond the margins of the original wound and invade the surrounding normal skin in a way that is site specific, and often contain whorls of collagen arranged in an abnormal fashion.

In contrast, normal skin consists of collagen fibers arranged in a basket-weave pattern, which contributes to both the strength and elasticity of the dermis. Thus, to achieve a smoother wound healing process, an approach is needed that not only stimulates collagen production, but also does so in a way that reduces scar formation.

The thermosetting biophotonic compositions of the present disclosure promote the wound healing by promoting the formation of substantially uniform epithelialization; promoting collagen synthesis; promoting controlled contraction; and/or by reducing the formation of scar tissue. The thermosetting biophotonic compositions of the present disclosure may promote wound healing by promoting the formation of substantially uniform epithelialization. The thermosetting biophotonic compositions of the present disclosure may promote collagen synthesis. The thermosetting biophotonic compositions of the present disclosure may promote controlled contraction. The thermosetting biophotonic compositions and methods of the present disclosure may promote wound healing, for example, by reducing the formation of scar tissue.

The thermosetting biophotonic compositions of the present disclosure may also be used in combination with negative pressure assisted wound closure devices and systems.

The thermosetting biophotonic compositions may be kept in place for up to one, two or 3 weeks, and illuminated with light which may include ambient light at various intervals. In this case, the composition may be covered up in between exposure to light with an opaque composition or left exposed to light.

*(6) Kits*

The present disclosure also provides kits for preparing a thermosetting biophotonic composition and/or providing any of the components required for forming thermosetting biophotonic compositions of the present disclosure.

The kit may include containers comprising the components or compositions that can be used to make the thermosetting biophotonic compositions of the present disclosure. The kit may include a thermosetting biophotonic composition of the present disclosure. The different components making up the biophotonic compositions of the present disclosure may be provided in separate containers. For example, the block copolymer may be provided in a container separate from the chromophore. Examples of such containers are dual chamber syringes, dual chamber containers with removable partitions, sachets with pouches, and multiple- compartment blister packs. Another example is one of the components being provided in a syringe which can be injected into a container of another component. In some embodiments, the composition is provided in a spray can or bottle.

The kit may also comprise a systemic drug for augmenting the treatment of the thermosetting biophotonic compositions of the present disclosure. For example, the kit may include a systemic or topical antibiotic, hormone treatment (e.g. for acne treatment or wound healing), or a negative pressure device.

The kit may also comprise a means for applying the components of the thermosetting biophotonic compositions of the disclosure.

In certain aspects, there is disclosed a container comprising a chamber for holding a thermosetting biophotonic composition, and an outlet in communication with the chamber for discharging the biophotonic composition from the container, wherein the thermosetting biophotonic composition comprises at least one chromophore solubilized in a block copolymer. The chamber may be partitioned to separate ingredients which are unstable on mixing, such as an oxidant and the chromophore. The container may further comprise a light source for activating the composition once discharged.

In certain embodiments of the kit, the kit may further comprise a light source such as a portable light with a wavelength appropriate to activate the chromophore of the thermosetting biophotonic composition. The portable light may be battery operated or re- chargeable.

Written instructions on how to use the thermosetting biophotonic compositions in accordance with the present disclosure may be included in the kit, or may be included on or associated with the containers comprising the compositions or components making up the thermosetting biophotonic composition of the present disclosure.

Identification of equivalent thermosetting biophotonic compositions, methods and kits are well within the skill of the ordinary practitioner and would require no more than routine experimentation, in light of the teachings of the present disclosure.

## EXAMPLES

### Example 1 - Preparation of an exemplary thermosetting biophotonic composition

A thermosetting composition was made, according to an embodiment of the present disclosure, comprising a poloxamer matrix incorporating therein chromophores. Specifically, Pluronic® F-127 was used which is a block copolymer of polyethylene glycol (PEG) and polypropylene glycol (PPG) (having a repeating unit comprising a PPG block linked to two PEG blocks), at a weight percentage which would allow it to thermoset to a cohesive biophotonic composition at temperatures between about 20°C and 39°C, e.g. when applied to a tissue or when applied to a tissue and/or heated with a lamp.

The thermosetting (gelation) behaviour of 20% w/v and 25 % w/v Pluronic F-127 solutions at different temperatures was evaluated. The solutions were prepared by dissolving Pluronic F-127 in cold de-ionised water (~ 4°C). For 20% w/v Pluronic solutions, 20g of the Pluronic F-127 powder was dissolved in 100 mL of cold water. For 25% w/v Pluronic F-127 solutions, 25g of the Pluronic F-127 powder was dissolved in 100 mL of cold water. The concentration of Pluronic F-127 is expressed in weight per volume of water. Gelation was evaluated by placing 2mL aliquots of the cold Pluronic F-127 solutions into test tubes and immersing the test tubes in water baths preheated to a well-defined temperature. Gelation was considered to have occurred if no flow was observed in the composition when the tubes were inverted. The results are summarized in Table 1.

*Table 1 - Thermosetting behaviour of Pluronic® F-127 at different concentrations.*

| | Temperature (°C) | | | |
|---|---|---|---|---|
| | **22** | **25** | **28** | **32** |
| 25% w/v Pluronic solution | Gelled in about 5 min | Gelled in about 1 min | Gelled in less than about 1 min | Gelled in less than about 1 min |
| 20% w/v Pluronic solution | No gelling | No gelling after about 20 mins | Gelled in about 5-6 mins | Gelled in about 1 min |

Next, the effect of carbamide peroxide on the gelation temperature of a 25% pluronic composition including a chromophore was evaluated. The presence of carbamide peroxide, at least at the concentration of 25%, appears to affect the gelation behaviour by increasing the temperature at which gelation occurs, but is well within a physiologically relevant temperature range. So these biophotonic compositions based on Pluronic F-127 can also include carbamide peroxide or other peroxides or peroxide precursors.

*Table 2 - Exemplary cohesive biophotonic compositions according to the present disclosure*

| | Pluronic Gel (g) | Carbamide (g) | Chromophore | Gelation Temp (°C) |
|---|---|---|---|---|
| Gel 1 | 8.80 | 1.20 (12 wt%) | 1.09 mg eosin Y (0.011 wt%) | 28 |
| Gel 2 | 10.00 | 0.00 | 1.09 mg eosin Y | 22 |
| Gel 3 | 10.00 | 0.00 | 1.09 mg eosin Y + 1.09 mg fluorescein (0.01 wt% eosin Y + 0.01 wt% fluorescein) | 22 |

It is worth noting that once gelled, all the pluronic solutions of Table 2 formed a transparent/translucent cohesive composition (not peelable). These cohesive biophotonic compositions could be made peelable by adding thickening agents such as cellulosic compositions, e.g. alginate, methyl cellulose, hydroxyethylcellulose or carboxymethylcellulose, or the like. The thermosetting was reversible in that thermogelled composition could re-liquefy if the temperature was reduced to below its gelling temperature.

The solutions of Table 2 were placed into pump-sprays and could be sprayed onto a target tissue to form a gel on touching the target tissue (at around 37°C). They could be removed easily by washing or wiping. Alternatively, a cold absorbent composition could be placed on the gel to bring the gel back to liquid form and soak it into the composition. The composition may be a sponge or a cloth. The composition needs only to be at a temperature below the gelling temperature of the gel.

When activated by blue light, the gels 1-3 of Table 2 absorbed and emitted light.

It will be clear to a skilled person that any other poloxamer can be used instead of Pluronic F-127 of this example, for example, P-123, L-122, L-61, L-121 and P-65, at weight percentages which would allow their gelation at or around body temperature, at or around skin temperature or lower.

**Example 2 - Biophotonic evaluation of the thermosetting biophotonic composition of Example 1**

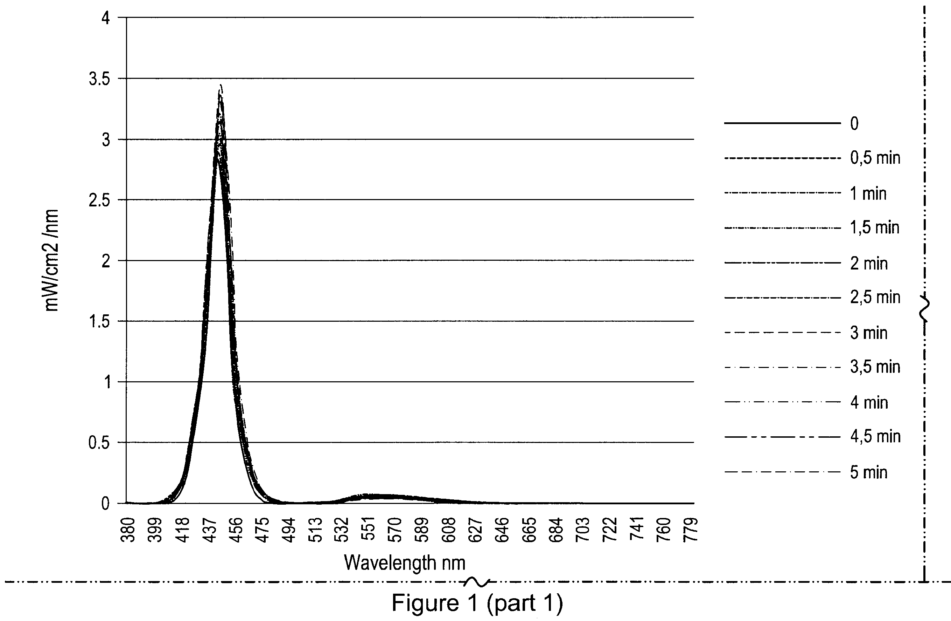

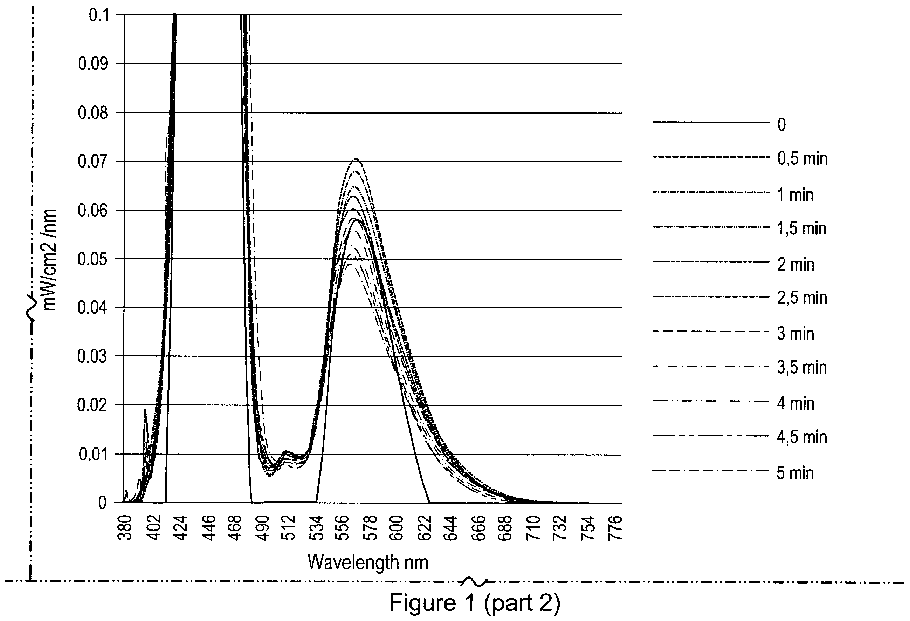

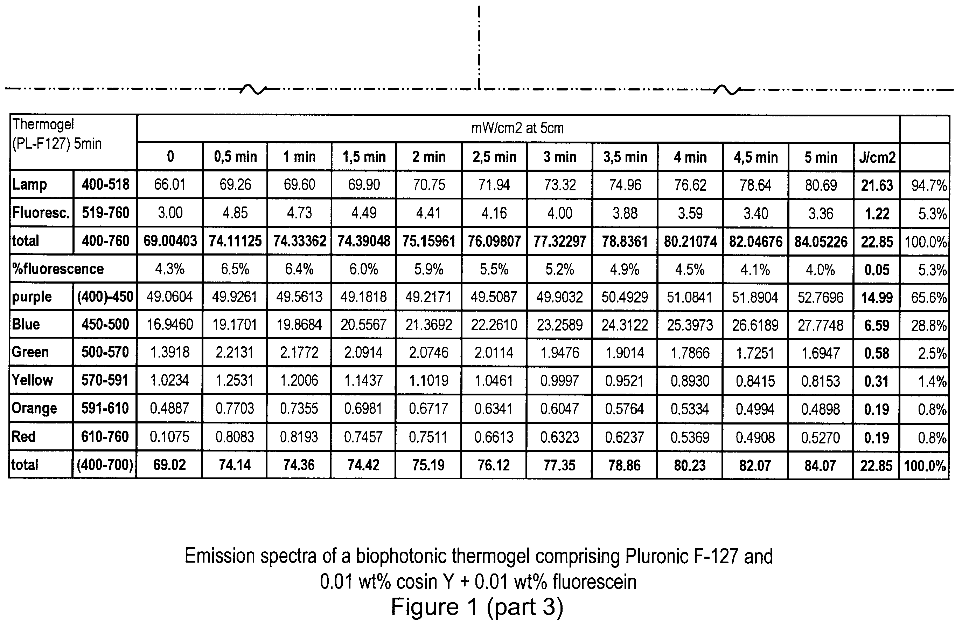

The emission spectra of gel 3 (comprising 0.01 wt% eosin Y + 0.01 wt% fluorescein) of Example 1 is shown in Figure 1. The emitted fluorescence was measured using a SP-100 spectroradiometer (SP-100, ORB Optronix) when illuminated for 5 minutes with a light having a peak wavelength of about 400-470 nm and a power density of about 30-150 mW/cm$^2$. As can be seen in Fig. 1, the chromophores did not fully photobleach after 10 minutes of illumination.

**Example 3 - Modulation of IL6 and IL8 by HaCaT human keratinocytes by the thermosetting biophotonic composition of Example 2**

The thermosetting biophotonic composition of Example 2 was evaluated for its ability to modulate inflammation, specifically cytokines IL6 and IL8. HaCaT human keratinocyte cells were used as an accepted in vitro model for assessing modulation of these inflammatory cytokines. Excessive, uncontrolled inflammation is observed in many skin conditions as well as in wounds, and can be detrimental to a host such as by impairing wound healing processes. Therefore a down regulation of IL6 and IL8 secretion may be beneficial in wound healing as well as alleviating other conditions, such as eczema and psoriasis.

A non-toxic concentration of IFNy was used to modulate the secretion of IL6 and IL8 by the HaCaT cells. Dexamethasone (final concentration of 5uM) was used as a positive control (strong inhibitor of pro-inflammatory cytokine production). The potential toxic effect of light on HaCaT cells was assessed using an *in vitro* toxicology assay kit, XTT based, which is a spectrophotometric evaluation of viable cell number.

Cell cultures were illuminated with light emitted by and transmitted through the thermosetting biophotonic composition of Example 2. The thermosetting biophotonic composition was positioned 5 cm above the cell cultures and illuminated with blue light having a peak wavelength between 400-470nm (average 460nm) and a power density of about 30-150 mW/cm$^2$ for 90 seconds.

Cytokine quantification was performed by cytokine ELISA on the culture supernatant 24 hours after illumination according to manufacturer instructions (DuoSet ELISA development kit from R&D Systems). The quantity of cytokine secreted was normalized to cell viability. No toxic effect was observed for all the test samples as measured by cell viability using a spectrophotometric evaluation of viable cell number 24 hours after treatment. All samples were screened in quadruplets. Three biological repetitions were performed for the tested membrane.

It was found that the light emitted by the thermosetting biophotonic composition of Example 2 produced a downward modulation of IL6 and IL8 on the IFNγ stimulated HaCaT cells. Table 3 summarizes the light treatment being received by the cultured cells during the illumination time from the thermosetting biophotonic composition, together with the IL6 and IL8 expression after illumination.

*Table 3. Light treatment being received by the cultured HaCaT cells during the illumination time from the thermosetting biophotonic composition and the IL6 and IL8 modulation observed.*

| | J/cm2, for 90 secondes exposure, THERA lamp at 5 cm | | | | | | HaCaT I FNg activated | |
| --- | --- | --- | --- | --- | --- | --- | --- | --- |
| | Violet | Blue | Green | Yellow | Orange | | Decrease **of** IL6 | Decrease of IL8 |
| THERM0GEL (PL-F127) E+F 0.011% each | 4.45 | 1.72 | 0.18 | 0.1 | 0.06 | 0.051 | 44%* | 36%' |
| Note: * statistically significant | | | | | | | | |

## Example 4 - Modulation of inflammation by the thermosetting biophotonic composition of Example 2

In order to gain more detailed picture of the biological effect mediated by the thermosetting biophotonic composition of Example 2, a Human Cytokine Antibody Array (RayBio C-Series, RayBiotech, Inc.) was performed. Cytokines are broadly defined as secreted cell-cell signaling proteins and play important roles in inflammation, innate immunity, apoptosis, angiogenesis, cell growth and differentiation. Simultaneous detection of multiple cytokines provides a powerful tool to study cell activity. Regulation of cellular processes by cytokines is a complex, dynamic process, often involving multiple proteins. Positive and negative feedback loops, pleiotrophic effects and redundant functions, spatial and temporal expression of or synergistic interactions between multiple cytokines, even regulation via release of soluble forms of membrane-bound receptors, are all common mechanisms modulating the effects of cytokine signaling.

The effect of light being transmitted through and from the thermosetting biophotonic composition on cytokine secretion profile in the culture medium by HaCaT cells was determined using Human Cytokine antibody Array (RayBio C-series from Raybiotech). In brief, HaCaT cells were illuminated as in Example 3. Supernatants were collected 24h post-illumination and incubated with arrayed antibody membranes according to the manufacturer instructions. Obtained signals were quantified with ImageJ software. For each experiment, the XTT assay was performed to normalize the quantity of cytokine secreted to the cell viability (cell viability was over 90% in all samples). All samples were done in quadruplets. The summary table below shows that treatment of the cells with thermosetting biophotonic composition can modulate the expression of proteins.

*Table 4. Modulation of protein expression in IFNg stimulated HaCaT cells 24 hours after treatment with THERA lamp and thermosetting biophotonic composition compared to control untreated cells.*

| **Cytokines** | |
| --- | --- |
| IL2 | - |
| IL3 | - |
| IL4 | ↓↓ |
| IL6 | ↓↓↓ |
| IL8 | - |
| IL10 | - |
| IL12 p40/70 | - |
| IL13 | - |
| IL15 | - |

(continued)

| Cytokines | |
|---|---|
| TNFalpha | ↓ |
| TNFbeta | - |
| IL1alpha | - |
| IL1beta | - |
| IFNgamma | - |
| | |
| | |
| MCP1 | - |
| MCP2 | - |
| MCP3 | ↓↓ |
| M-CSF | - |
| MDC | - |
| MIG | - |
| MIP-1delat | - |
| RANTES | - |
| TARC | ↓ |
| | |
| **Growth Factors** | |
| EGF | ↓ |
| IGF-1 | ↓ |
| ANG | ↓↓ |
| VEGF | ↓↓ |
| PDGF-BB | - |
| ENA-78 | - |
| G-CSF | - |
| GM-CSF | - |
| GRO | - |
| GROalpha | - |
| TGFbeta1 | - |
| Leptin | - |
| **↓ less than 25% decrease ↑ less than 25% increase** <br> **↓↓ 25-50% decrease ↑↑ 25-50% increase** <br> **↓↓↓ more than 50% decrease ↑↑↑ more than 50% increase** | |

CONCLUSIONS

The thermosetting biophotonic composition of Example 2 allowed blue light penetration (up to 6 J/cm$^2$ of energy fluency delivered to the cells) and produced fluorescence within the green and yellow light spectrum (up to 0.3 J/cm$^2$ of energy fluency delivered to cells). The results revealed that this light can downregulate pro-inflammatory cytokines IL-6 and IL-8 in HaCaT cells.

The protein array assay showed that the thermosetting biophotonic composition of Example 2 can also negatively modulate pro-inflammatory cytokines (such as TNF alpha, IL6) and pro-inflammatory chemokines (such as MCP-3, TARC) production. Interestingly, the thermosetting biophotonic composition could also downmodulate growth factors secretion (such as EGF, IGF-1, ANG, VEGF).

Therefore certain embodiments of the thermosetting biophotonic composition of the present disclosure may be useful in the inflammatory phase of wound healing, when fast resolution of this phase is desired, or in other inflammatory disorders.

**Example 5. Modulation of collagen production by thermosetting biophotonic composition**

Human Dermal Fibroblasts (DHF) cells were used here as an *in vitro* model to study the effect of visible blue light in combination with a thermosetting biophotonic composition of the present disclosure to evaluate the effect on the secretion of collagen, a component of the extracellular matrix.

Collagen production may be useful, for example, in wound healing, as well as for other indications such as skin conditions and rejuvenation. In wound healing, within four-five days upon injury, matrix-generating cells i.e. fibroblasts, move into the granulation tissue. These fibroblasts degrade the provisional matrix via MMPs and respond to cytokine/growth factors by proliferating and synthesizing new extracellular matrix (ECM) which is composed of collagen I, III, and V, proteoglycans, fibronectin and other components. TGF-beta concurrently inhibits proteases while enhancing protease inhibitors, favoring matrix accumulation.

A non-toxic concentration of TGFβ-1 was added to the cells to mimic hyperproliferation conditions. The potential toxic effect of light on HaCaT cells was assessed using an *in vitro* toxicology assay kit, XTT based, which is a spectrophotometric evaluation of viable cell number.

Cell cultures were illuminated with light emitted by and transmitted through the thermosetting biophotonic composition of Example 2. The gel was positioned 5 cm above the cell cultures and illuminated with blue light having a peak wavelength between 400-470nm and a power density of about 30-150 mW/cm$^2$ for 5 minutes. Vitamin C and TGFB1 was used as a positive control.

Forty eight hours after treatment, collagen production was evaluated using the picro-sirius red method. In brief, collagen molecules being rich in basic aminoacids strongly react with acidic dyes. Sirius red is an elongated dye molecule which reacts with collagen (type I, II, V), binds to it and after several washes which remove free dye the bounded Sirius red is eluted with sodium hydroxide and quantified using a spectrophotometer. All samples were screened in quadruplets. Two biological repetitions were performed for each of tested matrices.

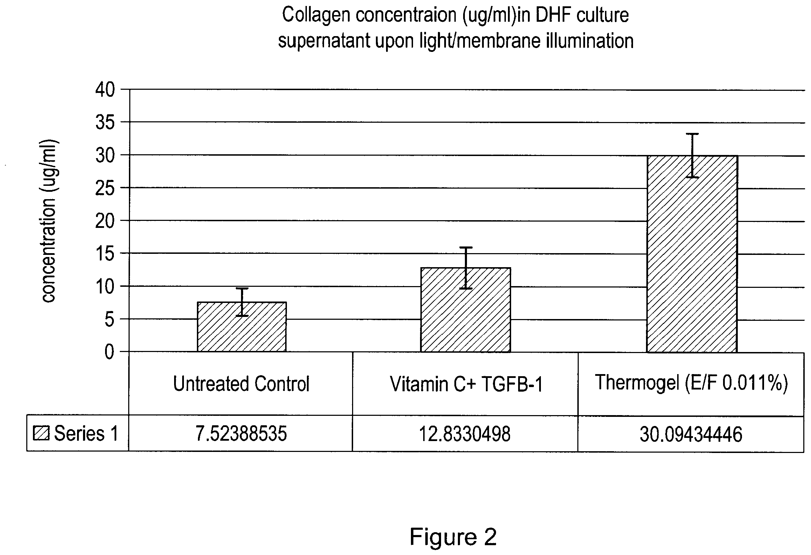

It can be seen from Figure 2, that light illumination through and by the thermosetting biophotonic composition of Example 2 can stimulate collagen production. A 4-fold increase in collagen production in DHF cell culture supernatant was observed compared to the untreated control.

**Claims**

1. A thermosetting biophotonic composition comprising a block copolymer at a concentration of more than 20% weight per volume of the composition, the block copolymer comprising at least one sequence of polyethylene glycol-propylene glycol (PEG-PPG), polyethylene glycol-poly(lactic acid) (PEG-PLA) or polyethylene glycol-poly(ε-caprolactone) (PEG-PCL), and at least one xanthene dye selected from Eosin Y, Erythrosine B, Fluorescein, Rose Bengal and Phloxin B, solubilized within the block copolymer, the at least xanthene dye being from 0.001 % to 3% per weight of the composition, the thermosetting biophotonic composition being in liquid form at 22°C and thermosetting on exposure of heat.

2. The thermosetting biophotonic composition of claim 1, wherein the block copolymer comprises at least one sequence of polyethylene glycol-propylene glycol (PEG-PPG).

3. The thermosetting biophotonic composition of claim 1, wherein the block copolymer is a poloxamer.

4. The thermosetting biophotonic composition of any one of claims 1-3, further comprising a stabilizer selected from gelatin, hydroxyethyl cellulose (HEC), carboxymethyl cellulose (CMC) and a thickening agent.

5. The thermosetting biophotonic composition of claim 1, further comprising an oxidizing agent, wherein the oxidizing agent is selected from hydrogen peroxide, urea peroxide and benzoyl peroxide.

6. A method for cosmetic treatment of tissue, the method comprising:

applying thermosetting biophotonic composition of any one of claims 1-5 over a target skin tissue; and illuminating the thermosetting biophotonic composition with light having a wavelength that overlaps with the absorption spectrum of the at least one xanthene dye.

7. The method of claim 6, wherein the cosmetic treatment of tissue is selected from skin rejuvenation, skin conditioning and promotion of collagen synthesis

8. The thermosetting biophotonic composition of any one of claims 1-5, for use in one of more of

- treating a skin disorder; and
- promoting would healing.

9. The thermosetting biophotonic composition for use of claim 8, wherein the skin disorder is selected from acne, eczema, psoriasis and dermatitis.

**Patentansprüche**

1. Wärmehärtende biophotonische Zusammensetzung, umfassend ein Blockcopolymer in einer Konzentration von mehr als 20 Gew.-% pro Volumen der Zusammensetzung, wobei das Blockcopolymer mindestens eine Sequenz von Polyethylenglycol-Propylenglycol (PEG-PPG), Polyethylenglycol-Poly(milchsäure) (PEG-PLA) oder Polyethylenglycol-Poly($\varepsilon$-Caprolacton) (PEG-PCL) und mindestens einen Xanthenfarbstoff umfasst, ausgewählt aus Eosin Y, Erythrosin B, Fluoreszein, Rose Bengal und Phloxin B, innerhalb des Blockcopolymers gelöst, wobei der mindestens Xanthenfarbstoff von 0,001 Gew.-% nach 3 Gew.-% der Zusammensetzung beträgt, wobei die wärmehärtende biophotonische Zusammensetzung bei 22 °C in flüssiger Form vorliegt und bei Hitzeeinwirkung wärmehärtend ist.

2. Wärmehärtende biophotonische Zusammensetzung nach Anspruch 1, wobei das Blockcopolymer mindestens eine Sequenz von Polyethylenglycol-Propylenglycol (PEG-PPG) umfasst.

3. Wärmehärtende biophotonische Zusammensetzung nach Anspruch 1, wobei das Blockcopolymer ein Poloxamer ist.

4. Wärmehärtende biophotonische Zusammensetzung nach einem der Ansprüche 1-3, weiter umfassend einen Stabilisator, ausgewählt aus Gelatine, Hydroxyethylcellulose (HEC), Carboyxemethylcellulose (CMC) und einem Verdickungsmittel.

5. Wärmehärtende biophotonische Zusammensetzung nach Anspruch 1, weiter umfassend ein Oxidationsmittel, wobei das Oxidationsmittel ausgewählt ist aus Wasserstoffperoxid, Harnstoffperoxid und Benzoylperoxid.

6. Verfahren zur kosmetischen Behandlung von Gewebe, wobei das Verfahren umfasst:

Auftragen von wärmehärtender biophotonischer Zusammensetzung nach einem der Ansprüche 1-5 über einem Zielhautgewebe; und
Bestrahlen der wärmehärtenden biophotonischen Zusammensetzung mit Licht, das eine Wellenlänge aufweist, die das Absorptionsspektrum des mindestens einen Xanthenfarbstoffs überlagert.

7. Verfahren nach Anspruch 6, wobei die kosmetische Behandlung von Gewebe ausgewählt ist aus Hautverjüngung, Hautpflege und Fördern von Kollagensynthese.

8. Wärmehärtende biophotonische Zusammensetzung nach einem der Ansprüche 1-5, zur Verwendung bei einem oder mehreren von

- Behandlung einer Hauterkrankung; und
- Fördern von Wundheilung.

9. Wärmehärtende biophotonische Zusammensetzung zur Verwendung nach Anspruch 8, wobei die Hauterkrankung ausgewählt ist aus Akne, Ekzem, Psoriasis und Dermatitis.

**EP 3 151 860 B1**

**Revendications**

1. Composition biophotonique thermodurcissable comprenant un copolymère bloc à une concentration supérieure à 20 % en poids par volume de la composition, le copolymère bloc comprenant au moins une séquence de polyéthylène glycol-propylène glycol (PEG-PG), polyéthylène glycol-acide polylactique (PEG-APL) ou polyéthylène glycol-poly($\varepsilon$-caprolactone) (PEG-PCL), et au moins un colorant xanthénique choisi parmi l'éosine Y, l'érythrosine B, la fluorescéine, le rose Bengale et la phloxine B, solubilisés dans le copolymère bloc, l'au moins colorant xanthénique étant de 0,001 % à 3 % en poids de la composition, la composition biophotonique thermodurcissable étant sous forme liquide à 22 °C et thermodurcissable lors de l'exposition à la chaleur.

2. Composition biophotonique thermodurcissable selon la revendication 1, dans laquelle le copolymère bloc comprend au moins une séquence de polyéthylène glycol-propylène glycol (PEG-PG).

3. Composition biophotonique thermodurcissable selon la revendication 1, dans laquelle le copolymère bloc est un poloxamère.

4. Composition biophotonique thermodurcissable selon l'une quelconque des revendications 1 à 3, comprenant en outre un stabilisateur choisi parmi la gélatine, l'hydroxyéthylcellulose (HEC), la carboxyméthylcellulose (CMC) et un agent épaississant.

5. Composition biophotonique thermodurcissable selon la revendication 1, comprenant en outre un agent oxydant, dans laquelle l'agent oxydant est choisi parmi le peroxyde d'hydrogène, le peroxyde d'urée et le peroxyde de benzoyle.

6. Procédé de traitement cosmétique des tissus, le procédé comprenant :

   l'application de la composition biophotonique thermodurcissable selon l'une quelconque des revendications 1 à 5 sur un tissu cutané cible ; et
   l'éclairage de la composition biophotonique thermodurcissable avec de la lumière présentant une longueur d'onde qui chevauche le spectre d'absorption de l'au moins un colorant xanthénique.

7. Procédé selon la revendication 6, dans lequel le traitement cosmétique des tissus est choisi parmi un rajeunissement de la peau, un soin de la peau et une stimulation de la synthèse de collagène.

8. Composition biophotonique thermodurcissable selon l'une quelconque des revendications 1 à 5 destinée à une utilisation dans l'un ou plusieurs parmi

   - le traitement d'une affection cutanée ; et
   - la stimulation de la cicatrisation des plaies.

9. Composition biophotonique thermodurcissable destinée à une utilisation selon la revendication 8, dans laquelle l'affection cutanée est choisie parmi l'acné, l'eczéma, le psoriasis et la dermatite.

Figure 1 (part 1)

Figure 1 (part 2)

| Thermogel (PL-F127) 5min | | mW/cm2 at 5cm | | | | | | | | | | | |
|---|---|---|---|---|---|---|---|---|---|---|---|---|---|
| | | 0 | 0,5 min | 1 min | 1,5 min | 2 min | 2,5 min | 3 min | 3,5 min | 4 min | 4,5 min | 5 min | J/cm2 | |
| Lamp | 400-518 | 66.01 | 69.26 | 69.60 | 69.90 | 70.75 | 71.94 | 73.32 | 74.96 | 76.62 | 78.64 | 80.69 | 21.63 | 94.7% |
| Fluoresc. | 519-760 | 3.00 | 4.85 | 4.73 | 4.49 | 4.41 | 4.16 | 4.00 | 3.88 | 3.59 | 3.40 | 3.36 | 1.22 | 5.3% |
| total | 400-760 | 69.00403 | 74.11125 | 74.33362 | 74.39048 | 75.15961 | 76.09807 | 77.32297 | 78.8361 | 80.21074 | 82.04676 | 84.05226 | 22.85 | 100.0% |
| %fluorescence | | 4.3% | 6.5% | 6.4% | 6.0% | 5.9% | 5.5% | 5.2% | 4.9% | 4.5% | 4.1% | 4.0% | 0.05 | 5.3% |
| purple | (400)-450 | 49.0604 | 49.9261 | 49.5613 | 49.1818 | 49.2171 | 49.5087 | 49.9032 | 50.4929 | 51.0841 | 51.8904 | 52.7696 | 14.99 | 65.6% |
| Blue | 450-500 | 16.9460 | 19.1701 | 19.8684 | 20.5567 | 21.3692 | 22.2610 | 23.2589 | 24.3122 | 25.3973 | 26.6189 | 27.7748 | 6.59 | 28.8% |
| Green | 500-570 | 1.3918 | 2.2131 | 2.1772 | 2.0914 | 2.0746 | 2.0114 | 1.9476 | 1.9014 | 1.7866 | 1.7251 | 1.6947 | 0.58 | 2.5% |
| Yellow | 570-591 | 1.0234 | 1.2531 | 1.2006 | 1.1437 | 1.1019 | 1.0461 | 0.9997 | 0.9521 | 0.8930 | 0.8415 | 0.8153 | 0.31 | 1.4% |
| Orange | 591-610 | 0.4887 | 0.7703 | 0.7355 | 0.6981 | 0.6717 | 0.6341 | 0.6047 | 0.5764 | 0.5334 | 0.4994 | 0.4898 | 0.19 | 0.8% |
| Red | 610-760 | 0.1075 | 0.8083 | 0.8193 | 0.7457 | 0.7511 | 0.6613 | 0.6323 | 0.6237 | 0.5369 | 0.4908 | 0.5270 | 0.19 | 0.8% |
| total | (400-700) | 69.02 | 74.14 | 74.36 | 74.42 | 75.19 | 76.12 | 77.35 | 78.86 | 80.23 | 82.07 | 84.07 | 22.85 | 100.0% |

Emission spectra of a biophotonic thermogel comprising Pluronic F-127 and
0.01 wt% cosin Y + 0.01 wt% fluorescein

Figure 1 (part 3)

Collagen concentraion (ug/ml)in DHF culture supernatant upon light/membrane illumination

| | Untreated Control | Vitamin C+ TGFB-1 | Thermogel (E/F 0.011%) |
|---|---|---|---|
| ☒ Series 1 | 7.52388535 | 12.8330498 | 30.09434446 |

Figure 2

**EP 3 151 860 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013155620 A
- US 20090069217

### Non-patent literature cited in the description

- **VAN HEMELRIJK.** *International Journal of Pharmaceutics,* 2011, vol. 437 (1-2), 120-129
- **DUMORTIER.** *Pharmaceutical Research,* 2006, vol. 23 (12), 2709-2728